# EUROPEAN PATENT SPECIFICATION

(11) **EP 4 096 526 B1**
(45) Date of publication and mention of the grant of the patent: **28.10.2026**
(21) Application number: 21705369.3
(22) Date of filing: 21.01.2021
(51) Int. Cl.: A61B 5/352, A61B 5/00, A61B 5/36, A61B 5/353, A61B 5/355, A61B 5/361, A61N 1/00

(54) **DEVICE AND METHOD FOR IDENTIFYING OVER-SENSED R-R INTERVALS**
VORRICHTUNG UND VERFAHREN ZUM IDENTIFIZIEREN ÜBERERFASSTER R-R-INTERVALLE
DISPOSITIF ET PROCÉDÉ POUR IDENTIFIER DES INTERVALLES R-R SURDÉTECTÉS

(30) Priority: 30.01.2020 US 202062967913 P; 20.01.2021 US 202117153036
(43) Date of publication of application: 07.12.2022
(73) Proprietor: PACESETTER, INC., Sylmar, CA 91342 (US)
(72) Inventor: BADIE, Nima, Sylmar, California 91342 (US); QU, Fujian, Sylmar, California 91342 (US); GILL, Jong, Sylmar, California 91342 (US)
(74) Representative: Barker Brettell Sweden AB
(86) International application number: PCT/US2021/014332
(87) International publication number: WO 2021/154578

(56) References cited:
- EP-A1- 3 566 746
- WO-A2-03/092810
- US-A1- 2015 045 682

## Description

### FIELD OF TECHNOLOGY

Embodiments described herein relate to analysis of an electrogram (EGM) or electrocardiogram (ECG), and more specifically, to identifying P-wave and T-wave oversensing, and more generally, to distinguishing true R-R intervals from over-sensed R-R intervals.

### BACKGROUND

Various types of implantable medical devices (IMDs) are used to monitor for cardiac arrythmias. Some types of IM Ds, such as implantable cardiac pacemakers and implantable cardiac defibrillators (1CDs), are capable of providing appropriate therapy in response to detected cardiac arrythmias. Other types of 1MDs, such as insertable cardiac monitors (ICMs), are used for diagnostic purposes. ICMs have been increasingly used to diagnose cardiac arrhythmias, particularly atrial fibrillation (AF).

Atrial Fibrillation (AF) is a very common type of supraventricular tachycardia (SVT) which leads to approximately one fifth of all strokes, and is the leading risk factor for ischemic stroke. However, AF is often asymptomatic and intermittent, which typically results in appropriate diagnosis and/or treatment not occurring in a timely manner. To overcome this, many cardiac devices, such as ICMs, now monitor for AF by obtaining an electrogram (EGM) signal and measuring R-R interval variability based on the EGM signal. For example, an 1CM or other IMD can compare measures of R-R interval variability to a variability threshold, to automatically detect AF when the variability threshold is exceeded. Indeed, ICMs predominantly identify AF by quantifying the variability in R-R intervals (i.e., by quantifying the variability in the timing of ventricular contractions).

There are a few ICMs that are commercially available, including the Confirm Rx^{™} ICM, manufactured by Abbott Laboratories, of Chicago, Illinois, the Reveal LINQ^{™} ICM, manufactured by Medtronic, Inc., of Minneapolis, Minnesota, and the BioMonitor^{™} 2 (AF and S versions), manufactured by Biotronik SE & Co. KG, of Berlin, Germany. When an ICM detects an episode of AF, information about the episode may be recorded and a corresponding EGM segment (and/or other information) can be transmitted from the 1CM to a patient care network for clinician review. False positive AF detections are highly undesirable, as the burden of sorting through large numbers of clinically irrelevant episodes of AF can be time consuming and costly.

In various IMDs, such as ICMs, clinicians are provided with the capability of programming an R-wave sensing threshold, to which samples of an EGM are compared to detect R-waves in the EGM, Clinicians often use relatively low R-wave sensing thresholds in order to reduce the undersensing of low amplitude R-waves. However, R-wave amplitudes may vary in real-life settings due to various reasons including physical activity and posture changes. If the R-wave sensing threshold is programmed to be too low (i.e., too sensitive), then lower amplitude features of the EGM (e.g. P-waves, T-waves, and/or noise) may be incorrectly sensed as R-waves. Unfortunately, false positive AF detections caused by oversensing higher-amplitude P-waves or T-waves (rather than just R-waves) are common, resulting in low AF detection specificity. Moreover, presently available P-wave and T-wave detection discriminator techniques are often unable or inadequate to correctly distinguish P-waves and T-waves from R-waves. Accordingly, there is a still a need for improved techniques for distinguishing P-waves and T-waves from R-waves, and for distinguishing over-sensed R-R intervals from true R-R intervals.

WO 03/092810 discloses a method and apparatus for automatically identifying various types of cardiac and non-cardiac oversensing. The apparatus comprises one or more electrodes, a sensing circuit coupled to the one or more electrodes and configured to obtain a signal indicative of electrical activity of a patient's heart. The apparatus also includes at least one of a processor or controller configured to obtain an ordered list of sensed intervals, based on the signal indicative of electrical activity of the patient's heart, identify true R-R intervals within the ordered list of sensed intervals, classify individual intervals as either a short interval or a long interval and identify one or more over-sensed R-R intervals based on an analysis of at least the short intervals.

For the avoidance of doubt, any embodiment, example or disclosure herein which would be regarded as a diagnostic method practiced on the human or animal body within the meaning of Article 53(c) EPC, in particular any method involving a diagnostic conclusion that a subject has or does not have AF, VT, SVT or another arrhythmia, is not part of the claimed invention.

### SUMMARY

The present invention is defined in the independent claims. Any disclosure herein not within the scope of the claims is presented as illustrative examples not forming part of the invention.

Certain embodiments of the present technology are directed to methods, devices, and systems that monitor HR and/or for one or more types of arrhythmic episodes based on sensed intervals that can include true R-R intervals as well as over-sensed R-R intervals. Such a method can include: obtaining an ordered list of sensed intervals, ordered in the order the intervals have been sensed, wherein the sensed intervals are determined between consecutive ones of potential R waves in a signal indicative of the electrical activity of the patient's heart, wherein each of the sensed intervals has a respective duration, wherein the ordered list of sensed intervals include true R-R intervals and one or more over-sensed R-R intervals due to T-wave and/or P-wave oversensing, wherein a true R-R interval is an interval between consecutive actual R-waves, and wherein an over-sensed R-R interval is a P-R interval between an over-sensed P-wave and a consecutive actual R-wave, a R-P interval between an actual R-wave and a consecutive over-sensed P-wave, a T-R interval between an over-sensed T-wave and a consecutive actual R-wave, or a R-T interval between an actual R-wave and a consecutive over-sensed T-wave; identifying an actual R-R interval as a true R-R interval within the ordered list of sensed intervals; removing, from the ordered list of sensed intervals, each of the sensed intervals that was identified as being a true R-R interval, to thereby produce a remaining ordered list of sensed intervals; classifying individual intervals in the remaining ordered list of sensed intervals as either a short interval when a duration of the interval is shorter than the duration of an immediately following interval and/or the duration of an immediately preceding interval in the remaining ordered list of sensed intervals, or a long interval when a duration of the interval is longer than the duration of an immediately following interval and/or the duration of an immediately preceding interval in the remaining ordered list of sensed intervals; and identifying sensed intervals in the remaining ordered list of sensed intervals as one or more over-sensed R-R intervals based on an analysis of at least the short intervals in the remaining ordered list of sensed intervals. The identifying true R-R intervals within the ordered list of sensed intervals comprises: identifying as a said true R-R interval, each said sensed interval having a duration that is within a first specified threshold of either (i) a sum of the durations of an immediately preceding two intervals in the ordered list of sensed intervals, or (ii) a sum of the durations of an immediately following two intervals in the ordered list of sensed intervals; and identifying as a said true R-R interval, each said sensed interval having a duration that is within a second specified threshold of a mean or median of the durations of the sensed intervals already identified as true R-R intervals, wherein the second specified threshold may or may not be the same as the first specified threshold. The identifying one or more over-sensed R-R intervals comprises, for at least one said sensed interval classified as a said short interval in the remaining ordered list of sensed intervals, identifying the sensed interval as an over-sensed R-R interval when all of the following are true: a difference between a duration of the sensed interval that was classified as a said short interval and a duration of an immediately preceding sensed interval that was classified as a said short interval is within a third specified threshold; a difference between the duration of the sensed interval that was classified as a said short interval and a duration of an immediately following sensed interval that was classified as a said long interval is greater than a fourth specified threshold; and the duration the sensed interval that was classified as a said short interval is less than a fifth specified threshold.

In accordance with certain embodiments, the method further comprises: determining an oversensing score based on how many of the sensed intervals classified as a said short interval were identified as being an over-sensed R-R interval; and determining that a detected AF episode or other type of arrhythmic episode was a false positive defection when the oversensing score exceeds a specified oversensing threshold. In accordance with certain embodiments, the specified oversensing threshold comprises a specified percentage threshold, the oversensing score comprises what percentage of all of the sensed intervals classified as a said short interval were identified as being an over-sensed R-R interval, and a detected AF episode or other type of arrhythmic episode is determined to be a false positive detection when the determined percentage exceeds the specified percentage threshold.

In accordance with certain embodiments, such a method is performed by an IMD in response to an AF episode or other type of arrhythmic episode being detected by the IMD, and the method further comprises the IMD determining, based on results of the steps summarized above, whether the AF episode or other type of arrhythmic episode that was detected by the IMD was a false positive detection. Such an IMD can be, for example, an insertable cardiac monitor (ICM), a cardiac pacemaker to which one or more leads is/are attached, a leadless cardiac pacemaker (LCP), or an implantable cardioverter defibrillator (ICD).

In accordance with certain embodiments, the IMD is configured to transmit, to an external device that is communicatively coupled to a patient care network, data corresponding to an AF episode or other type of arrhythmic episode that is detected by the IMD. In such an embodiment, the method can further comprise the IMD preventing the transmitting, to the external device that is communicatively coupled to the patient care network, data corresponding to an AF episode or other type of arrhythmic episode that is detected by the IMD but is thereafter determined by the IMD as being a false positive detection. Alternatively, data corresponding to a limited amount of AF episodes or other type of arrhythmic episodes that is/are detected by the IMD but is/are thereafter determined by the IMD as being a false positive defection can be transmitted to a patient care network so that such data can be used by a clinician to adjust an R-wave sensing threshold.

In accordance with certain embodiments the ordered list of sensed intervals is obtained by: obtaining an electrogram (EGM) segment corresponding to a period of time preceding and leading up to the AF episode or other type of arrhythmic episode that was detected; identifying potential R-waves within the EGM segment; and determining intervals between consecutive ones of the potential R-waves to thereby produce the ordered list of sensed intervals.

In accordance with certain embodiments, steps summarized above are performed by a device that monitors heart rate (HR) based on intervals Identified from a segment of an electrogram (EGM) or electrocardiogram (ECG), and the method further comprises: the device determining, based on results of steps summarized above, whether a monitored HR is inaccurate due to oversensing and thus should be ignored or recalculated.

In accordance with certain embodiments, the method further comprises, after identifying true R-R intervals and identifying over-sensed R-R intervals: combining pairs of over-sensed R-R intervals to identify further true R-R intervals; producing a corrected ordered list of sensed intervals that includes the true R-R intervals (initially identified without any combining) and includes the further true R-R intervals identified by combining pairs of over-sensed R-R intervals; and monitoring HR and/or for an arrhythmic episode based on the corrected ordered list of sensed intervals.

In accordance with certain embodiments, a device comprises: one or more electrodes; a sensing circuity coupled to the one or more electrodes and configured to obtain a signal indicative of electrical activity of the patient's heart; and at least one of a processor or controller. The at least one of a processor or controller is configure to: obtain an ordered list of sensed intervals, , ordered in the order the intervals have been sensed, wherein the sensed intervals are determined between consecutive ones of potential R-waves in the signal indicative of the electrical activity of the patient's heart, wherein each of the sensed intervals has a respective duration, wherein the ordered list of sensed intervals include true R-R intervals and one or more over-sensed R-R intervals due to T-wave and/or P-wave oversensing, wherein a true R-R interval is an interval between consecutive actual R-waves, and wherein an over-sensed R-R interval is a P-R interval between an over-sensed P-wave and a consecutive actual R-wave, a R-P interval between an actual R-wave and a consecutive over-sensed P-wave, a T-R interval between an over-sensed T-wave and a consecutive actual R-wave, or a R-T interval between an actual R-wave and a consecutive over-sensed T-wave; identify actual R-R intervals as a true R-R interval within the ordered list of sensed intervals. The at least one of a processor or controller is/are also configured to: remove, from the ordered list of sensed intervals, each of the sensed intervals that was identified as being a true R-R interval, to thereby produce a remaining ordered list of sensed intervals; classify individual intervals, in the remaining ordered list of sensed intervals, as either a short interval when a duration of the interval is shorter than the duration of an immediately following interval and/or the duration of an immediately preceding interval in the remaining ordered list of sensed intervals or a long interval when a duration of the interval is longer than the duration of an immediately following interval and/or the duration of an immediately preceding interval in the remaining ordered list of sensed intervals; and identify sensed intervals in the remaining ordered list of sensed intervals as one or more over-sensed R-R intervals based on an analysis of at least the short intervals in the remaining ordered list of sensed intervals, wherein in order to identify true R-R intervals within the ordered list of sensed intervals the at least one of a processor or controller is/are configured to identify as a said true R-R interval, each said sensed interval having a duration that is within a first specified threshold of either (i) a sum of the durations of an immediately preceding two intervals in the ordered list of sensed intervals, or (ii) a sum of the durations of an immediately following two intervals in the ordered list of sensed intervals; and identify as a said true R-R interval, each said sensed interval having a duration that is within a second specified threshold of a mean or median of the durations of the sensed intervals already identified as true R-R intervals, wherein the second specified threshold may or may not be the same as the first specified threshold. Wherein in order to identify a said sensed interval, that has been classified as a said short interval in the remaining ordered list of sensed intervals, as an over-sensed R-R interval, the at least one of a processor or controller is/are configured to identify the sensed interval as an over-sensed R-R interval when all of the following are true: a difference between a duration of the sensed interval that was classified as a said short interval and a duration of an immediately preceding sensed interval that was classified as a said short interval is within a third specified threshold; a difference between the duration of the sensed interval that was classified as a said short interval and a duration of an immediately following sensed interval that was classified as a said long interval is greater than a fourth specified threshold; and the duration the sensed interval that was classified as a said short interval is less than a fifth specified threshold. Such a device can be an ICM, a cardiac pacemaker to which one or more leads is/are attached, an LCP, or an ICD.

In accordance with certain embodiments, the at least one of a processor or controller is/are further configured to: determine an oversensing score based on how many of the sensed intervals classified as a said short interval were identified as being an over-sensed R-R interval; and determine, based on the oversensing score, whether a detected AF episode or other type of arrhythmic episode was a false positive detection.

In accordance with certain embodiments, the at least one of a processor or controller is/are further configured to: combine pairs of over-sensed R-R intervals to identify further true R-R intervals; produce a corrected ordered list of sensed intervals including true R-R intervals, at least some of which are identified by combining pairs of over-sensed R-R intervals; and monitor heart rate and/or for an arrhythmic episode based on the corrected ordered list of sensed intervals.

In accordance with certain embodiment, the device further comprises a transceiver configured to wireless communicate with an external device. Also, the at least one of a processor or controller is/are further configured to interact with the transceiver to: cause data corresponding to an AF episode or other type of arrhythmic episode that is detected by the device be selectively transmitted to the external device; and prevent data corresponding to an AF episode or other type of arrhythmic episode that is detected by the device, but is thereafter determined by the device as being a false positive detection, from being transmitted to the external device.

The present invention is defined by the appended claims.

### BRIEF DESCRIPTION OF THE DRAWINGS

FIG. 1 includes a portion of an EGM segment that resulted in an atrial AF detection due to over-sensed T-waves and includes a graph of HR versus time.
FIG. 2 includes a graph of sensed intervals for a window preceding an AF trigger_{;} which sensed intervals correspond to the inverse of the heart rates shown in FIG. 1.
FIG. 3 includes a left panel graph that is the same as FIG. 2, a center panel graph that illustrates how true R-R intervals can be calculated by adding durations of identified R-T and T-R intervals, and a right panel Poincare plot that illustrates actual R-R interval stability after oversensing correction of the present technology is performed.
FIGS. 4A, 4B, and 4C, which can be collectively referred to as FIG. 4, includes a high level flow diagram that is used to describe how true R-R intervals can be distinguished from over-sensed R-R intervals using embodiments of the present technology, and how an oversensing score can be determined and used.
FIG. 5 shows a block diagram of one embodiment of an IMD that is implanted into a patient in accordance with certain embodiments of the present technology.

### DETAILED DESCRIPTION

It is well known that each cardiac cycle represented within an EGM or ECG typically includes a P-wave, followed by a QRS complex, followed by a T-wave, with the QRS complex including Q-, R-, and S-waves. The P-wave is caused by depolarization of the atria. This is followed by atrial contraction, which is indicated by a slight rise in atrial pressure contributing to further filling of the ventricle. Following atrial contraction is ventricular depolarization, as indicated by the QRS complex, with ventricular depolarization initiating contraction of the ventricles resulting in a rise in ventricular pressure until it exceeds the pulmonary and aortic diastolic pressures to result in forward flow as the blood is ejected from the ventricles. Ventricular repolarization occurs thereafter, as indicated by the T-wave and this is associated with the onset of ventricular relaxation in which forward flow stops, the pressure in the ventricle falls below that in the atria at which time the mitral and tricuspid valves open to begin to passively fill the ventricle during diastole. The terms EGM, EGM signal, and EGM waveform are used interchangeably herein. Similarly, the terms ECG, ECG signal, and ECG waveform are used interchangeably herein. Both ECG and EGM signals are signals indicative of electrical activity of a patient's heart.

The R-wave is the largest wave in the QRS complex, and it often identified by comparing samples of an EGM or ECG to an R-wave threshold. Various measurements can be obtained based on the EGM or ECG waveform, including measurements of R-R intervals, where an R-R interval is the duration between a pair of consecutive R-waves. As noted above, in the Background, a common technique for detecting AF is based on measures of R-R interval variability. However, where T-waves and/or P-waves are falsely identified as R-waves, false R-R intervals can be identified which have a high variability, leading to false detections of AF. In other words, over-sensed P-waves and/or over-sensed T-waves can lead to false positive AF detections. An over-sensed P-wave, as the term is used herein, refers to a P-wave that is falsely identified as an R-wave. Similarly, an over-sensed T-wave, as the term is used herein, refers to a T-wave that is falsely identified as an R-wave. An under-sensed R-wave, as the term is used herein, refers to an R-wave that is not detected.

Certain embodiments of the present technology relate to methods and devices that use sensed intervals to determine whether P-wave and/or T-wave oversensing has occurred, and more generally, to distinguish true R-R intervals from false R-R intervals. These embodiments can beneficially be used, for example, to prevent or reject false positive AF detections before they are transmitted to a clinician, consequently improving AF detection specificity and reducing downstream clinical resources. A true R-R interval, as the term is used herein, refers to an actual R-R interval. A false R-R interval, as the term is used herein, refers to an interval that is mistakenly identified as an R-R interval, but is not an actual R-R interval. Exemplary types of intervals that may be mistakenly identified as an R-R interval, and thus are examples of false R-R intervals, include, but are not limited to, P-R intervals, R-T intervals, P-T intervals, and T-P intervals. A P-R interval can be mistakenly identified as an R-R interval where a P-wave is over-sensed. An R-T interval can be mistakenly identified as R-R interval where a T-wave is over-sensed. A P-T interval or a T-P interval can be mistakenly identified as an R-R interval where T- and P-waves are over-sensed and an R-wave is under-sensed. These are just a few examples of types of false R-R intervals and how they may occur, which examples are not intended to be all inclusive. False R-R intervals are also referred to herein as over-sensed R-R intervals.

Certain embodiments of the present technology described herein rely on the phenomenon that an over-sensed P-wave or an over-sensed T-wave effectively divides a normal R-R interval into two shorter intervals (e.g. an R-T interval and a T-R interval), the sum of which is an actual R-R interval, which can also be referred to as the true R-R interval. Thus, certain embodiments of the present technology identify intervals that are similar to the sum of either the last two intervals or the next two intervals, which are the real R-R intervals, and do not correspond to oversensing. Further analysis (e.g., arrythmia detection analysis) can then proceed using only the remaining intervals. Of the remaining R-R intervals, intervals are flagged if, looking at the next two intervals, they follow a "short-long-short" pattern, wherein the terms "short" and "long" are relative terms, as both groups of intervals are much shorter than true R-R intervals. Of these flagged intervals, an "oversensing score" can be calculated as the percentage of these intervals which are (i) sufficiently similar in duration to the neighboring flagged intervals, (ii) sufficiently different in duration from the neighboring (unflagged) intervals, and (iii) sufficiently short in duration. This "oversensing score" can be an estimate of the percentage of intervals that either start or end with an over-sensed R-wave. Explained another way, the "oversensing score" can be an estimate of the percentage of short intervals that classified as over-sensed R-R intervals. If the "oversensing score" in the window preceding an AF trigger exceeds a specified threshold (e.g. 50%), then the AF trigger can be associated with oversensing (i.e., false positive AF), and can be rejected. Further and alternative details of such embodiments of the present technology are described below.

In accordance with certain embodiments, a list of potential sensed R-R intervals is obtained for a recorded EGM clip, which can also be referred to as a segment of an EGM, or an EGM segment. Because this list of potential R-R intervals may actually including P-R intervals, R-P intervals, T-R intervals, and/or R-T intervals, due to oversensing of P-waves and/or T-waves, these potential R-R intervals will often be referred to more generally as "intervals" in this document for simplicity. While a large portion of the following description and patient example discussed below describes T-wave oversensing, the same principles apply to P-wave oversensing.

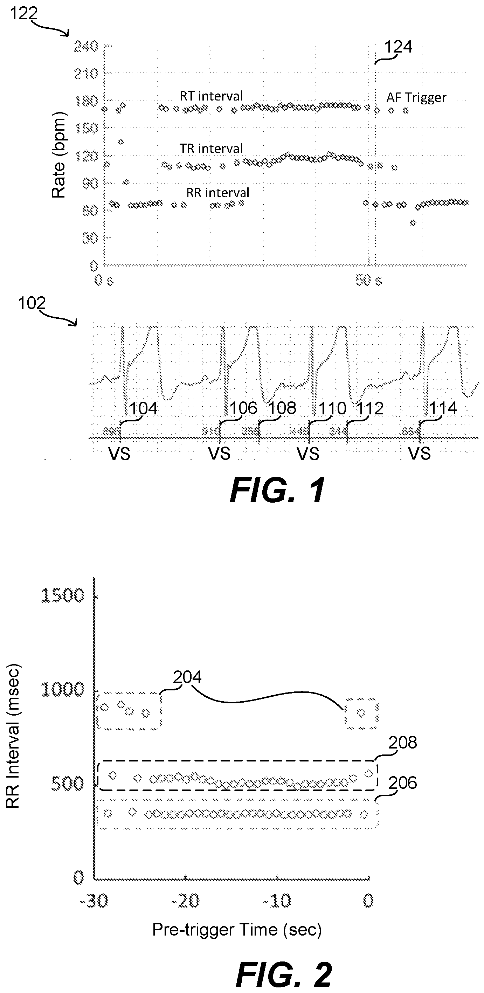

An example of T-wave oversensing is shown if FIG. 1, taken directly from an EGM clip file of a patient having an implanted Confirm RX^{™} ICM. At the bottom of FIG. 1 is shown a portion of an EGM segment 102 that resulted in an AF detection due to over-sensed T-waves. At the top of FIG. 1 is shown a graph or plot that includes heart rate (HR) in beats per minute (bpm) along the vertical axis, and time in seconds (s) long the horizontal axis. The dashed vertical line 124 corresponds to an AF detection occurring at a point in time corresponding to ~52 seconds, and thus, the vertical line 124 is also marked AF Trigger.

Referring to the portion of the EGM segment 102 shown at the bottom of FIG. 1, actual R-waves in the EGM segment 102 that correspond to actual ventricular sensed (VS) events are marked VS. Next to the first vertical line 104 marked VS is the number 895, referring to an R-R interval of 895 ms. Next to the second vertical line 106 marked VS is the number 910, referring to an R-R interval of 910 ms. Following the second vertical line 106 marked VS is another vertical line 108, which is not marked VS, and actually corresponds to an over-sensed T-wave. Next to the vertical line 108 is the number 355, referring to an R-T interval of 355 ms. Following the vertical line 108 is the vertical line 110 marked VS, which corresponds to a true R-wave. Next to the vertical line 110 is the number 445, referring to a T-R interval of 455 ms. Following the vertical line 110 is the vertical line 112, which corresponds to another over-sensed T-wave. Next to the vertical line 112 is the number 344, referring to an R-T interval of 344 ms. Following the vertical line 112 is the vertical line 114 marked VS, which corresponds to a true R-wave. Next to the vertical line 114 is the number 664, referring to a T-R interval of 664 ms. In summary, the ordered list of intervals obtained from the portion of the EGM segment 102 shown at the bottom of FIG. 1 have intervals of: 895 ms, 910 ms, 355 ms, 445 ms, 344 ms, and 664 ms.

Note that the interval between the vertical line labeled 106 and the vertical line labeled 110 (both marked VS, and thus corresponding to true R-waves) is 900 ms (i.e., 355 ms + 445 ms = 900 ms). Also note that the interval between the vertical line labeled 110 and the vertical line labeled 114 (both marked VS, and thus corresponding to true R-waves) is 1008 ms (i.e., 344 ms + 664 ms = 1008 ms). Accordingly, if the over-sensed T-waves (corresponding to the vertical lines 108 and 112) were not detected, then the ordered list of intervals obtained from the portion of the EGM segment 102 shown at the bottom of FIG. 1 would instead have intervals of: 895 ms, 910 ms, 900 ms, and 1008 ms.

In the example shown in FIG. 1, the true R-R intervals are associated with a HR of approximately 65 bpm. Thus, the true R-R intervals are approximately 923 milliseconds (ms), because 60 sec/min divided by 65 bpm = 0.923 seconds = 923 ms. However, where there are over-sensed T-waves, every over-sensed T-wave results in two shorter intervals with HRs of approximately 110-120 bpm (R-T intervals) and 170-175 bpm (T-R intervals). For an example, where there is an over-sensed T-wave detected between a pair of true R-waves, rather than the list of intervals including a true R-R interval (having a duration of 873 ms), the list can instead include an R-T interval of approximately 520 ms and a T-R interval of approximately 353 ms.

In accordance with certain embodiments, in order to simplify implementation, techniques for identifying P-wave and/or T-wave oversensing are based on intervals, rather than heart rates. However, it is noted that such techniques can alternatively be heart rate based, instead of intervals based, while still being within the scope of the embodiments of the present technology. For the following discussion, such techniques focus on a 30 second window preceding an AF trigger. However, it is noted that windows of other (i.e., longer or shorter) lengths can instead be used while still being within the scope of the embodiments described herein. The AF trigger can be detected in various different manners, such as by analyzing heart rate variability and/or R-R interval variability, but is not limited thereto.

FIG. 2 illustrates a graph or plot of sensed intervals (in ms) for a 30 ms window preceding an AF trigger, which sensed intervals correspond to the inverse of the heart rates that were shown in FIG. 1. In FIG. 2, the dashed blocks 204 corresponds to sensed intervals that are true R-R intervals, the dashed block 206 corresponds to the shorter of the two groups of over-sensed intervals, and the dashed block 208 corresponds to the longer of the two groups of over-sensed intervals. More specifically, the dashed block 206 corresponds to R-T intervals, and the dashed block 208 corresponds to the T-R intervals.

Where a T-wave is mistakenly detected as an R-wave, it can be said that T-wave oversensing occurred, or that an over-sensed R-wave was detected. As can be appreciated from FIGS. 1 and 2, with every over-sensed R-wave (resulted from T-wave oversensing), the true R-R interval is bisected into two shorter intervals, the sum of which is the true R-R interval. Similarly, where a P-wave is mistakenly detected as an R-wave, it can be said that P-wave oversensing occurred, or that an over-sensed R-wave was detected. If P-wave oversensing occurs (instead of T-wave oversensing), with every over-sensed R-wave (resulted from P-wave oversensing), the true R-R interval is also bisected into two shorter intervals, the sum of which is the true R-R interval.

Certain embodiments of the present technology identify intervals that are not true R-R intervals. In other words, certain embodiments of the present technology identify false R-R intervals, which can also be referred to as over-sensed R-R intervals. Examples of over-sensed R-R intervals include R-T intervals and T-R intervals, as can be appreciated from the above discussion of FIGS. 1 and 2. Other examples of over-sensed R-R intervals include P-R intervals and R-P intervals.

When attempting to identify over-sensed R-R intervals, it is useful to remove true R-R intervals from the list of intervals being analyzed. This can be accomplished by removing all intervals that are within a specified percentage (e.g., 10%) of the sum of either the immediately preceding two or the immediately following two intervals within the list, as such intervals are likely true R-R intervals with a neighboring over-sensed interval. However, depending on how frequently oversensing occurs, a true R-R may not be neighbored by over-sensed R-waves. Thus, R-R intervals are also classified at true R-R intervals and removed from the list if they are within a specified percentage (e.g., 10%) of the median or mean of the previously-removed "true" R-R intervals. Henceforth, the algorithm will rely only on the remaining intervals that were not removed (i.e., questionable R-R intervals) to identify over-sensed R-R intervals.

For the remaining intervals, oversensing would result in a consistent "short-long-short" alternation. In the case of T-wave oversensing, this pattern corresponds to a short R-T interval (ventricular repolarization time) followed by a slightly longer T-R interval (from ventricular repolarization until the next ventricular depolarization). In the case of P-wave oversensing, this pattern corresponds to a short P-R interval (atrioventricular conduction time) followed by a slightly longer R-P interval (from ventricular depolarization until the next atrial depolarization).

To quantify how often this "short-long-short" pattern occurs, each remaining interval is labeled or classified as either "short" (shorter than the next non-removed interval, which next non-removed interval is longer than the following non-removed interval) or "long" (longer than the next non-removed interval, which next non-removed interval is in turn shorter than the following non-removed interval). In other words, interval (i) < interval (i+1), and interval (i+1) > interval (i+2). Note that both the "short" and "long" intervals are both much shorter than the true R-R interval-they are "short" or "long" relative to each other. However, certain rhythms can also produce a short- long-short pattern by happenstance, such as premature atrial contraction and premature ventricular contractions (PACs and PVCs) or AF. Thus, in accordance with certain embodiments, three more discrimination criteria are used to count the number of "short" intervals associated with oversensing. In other words, these discrimination criteria are used to determine whether individual short intervals are over-sensed R-R intervals. Such three additional discrimination criteria, each of which are discussed below, include: short interval variability, short-long interval discrepancy, and short interval duration.

Short Interval Variability - First, during P-wave or T-wave oversensing, the "short" intervals (i.e., P-R interval or R-T interval) should have relatively consistent values, as they depend on either atrioventricular conduction times (P-R) or ventricular repolarization times (R-T). In contrast, the longer intervals (e.g. R-P or T-R) depend on the heart rate, which is much more variable than conduction times or repolarization times. Furthermore, AF or PVCs, which may also demonstrate a "short-long-short" pattern, would result in highly variable "short" interval durations. Therefore, the "short" intervals that truly correspond to oversensing should have low interval-interval variability, quantified by a difference from the previous "short" interval of less than 10%, or some other specified threshold.

Short-Long Interval Discrepancy - The second discrimination criterion serves to discriminate oversensing from R-wave undersensing. During R-wave undersensing, one under-sensed R-wave results in an R-R interval that is similar to the sum of the previous two or next two intervals, effectively appearing as a true R-R interval surrounded by intervals associated with oversensing. However, in the case of undersensing, the "short" and "long" intervals would be almost identical in duration. Therefore, the second discrimination criterion ensures a sufficient discrepancy between the "short" and "long" intervals. Specifically, the "short" intervals that truly correspond to oversensing should differ from the next "long" interval by greater than 5% (or some other specified threshold).

Short Interval Duration - The third discrimination criterion handles the possibility that all of the above criteria could be satisfied by slow, yet variable, heart rates. During true oversensing, however, the "short" intervals should correspond to a very fast heart rate. Thus, the third discrimination criterion requires that the "short" intervals should all have values less 444 ms (i.e., greater than 135 bpm), or some other specified threshold. If the over-sensed R-waves corresponding to these "short" intervals are eliminated, the true R-R intervals can be calculated.

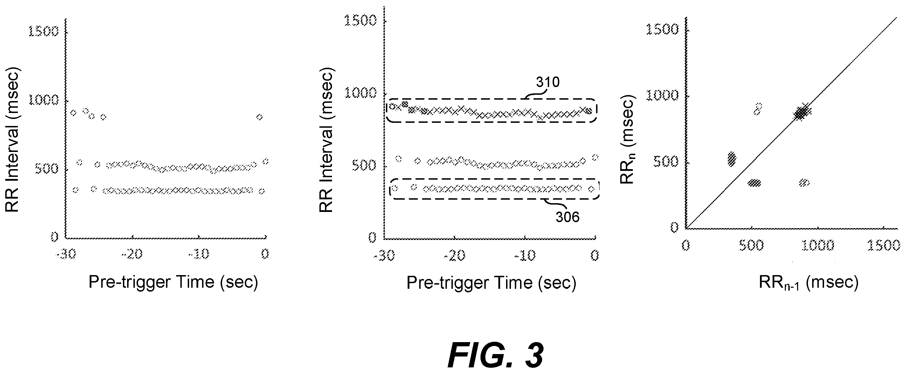

FIG. 3 illustrates how embodiments of the present technology can be used to impact the example intervals described with references to FIGS. 1 and 2. The left panel in FIG. 3, which is the same as FIG. 2 discussed above, includes true R-R intervals as well as over-sensed R-R intervals (which include R-T intervals and T-R intervals), as was explained above with reference to FIG. 2. Using embodiments of the present technology summarized above, and described in additional detail below, the original sensed intervals (shown in the left panel) are corrected by first removing true R-R intervals. Next, the true "short" over-sensed intervals (the lower horizontal line of circles within the dashed block 306 in the center panel of FIG. 3) are identified. By eliminating the over-sensed R-waves corresponding to these "short" intervals, the true R-R intervals that remain can be calculated, which are shown as Xs within the dashed block 310 (in the center panel of FIG. 3). More specifically, durations of the true R-R intervals represented by the Xs within the dashed block 310 (in the center panel of FIG. 3) are calculated by adding (aka summing) the durations of pairs of sensed intervals that include one short interval (an R-T interval) and one long interval (a T-R interval). Note that calculated (aka corrected) R-R intervals in the center panel in FIG. 3 are consistent with the previously identified and removed true R-R intervals. The Poincare plot (shown in the right panel in FIG. 3) plots the relationship between each interval and the immediately preceding interval, showing the same data and markers as the center panel. This Poincare plot illustrates the actual R-R interval stability (Xs), after oversensing correction of the original R-R intervals (circles) by the present technology is performed. Before oversensing correction, each interval differs, albeit predictably, from the preceding interval. After oversensing correction, all the interval-interval difference is negligible (overlapping Xs).

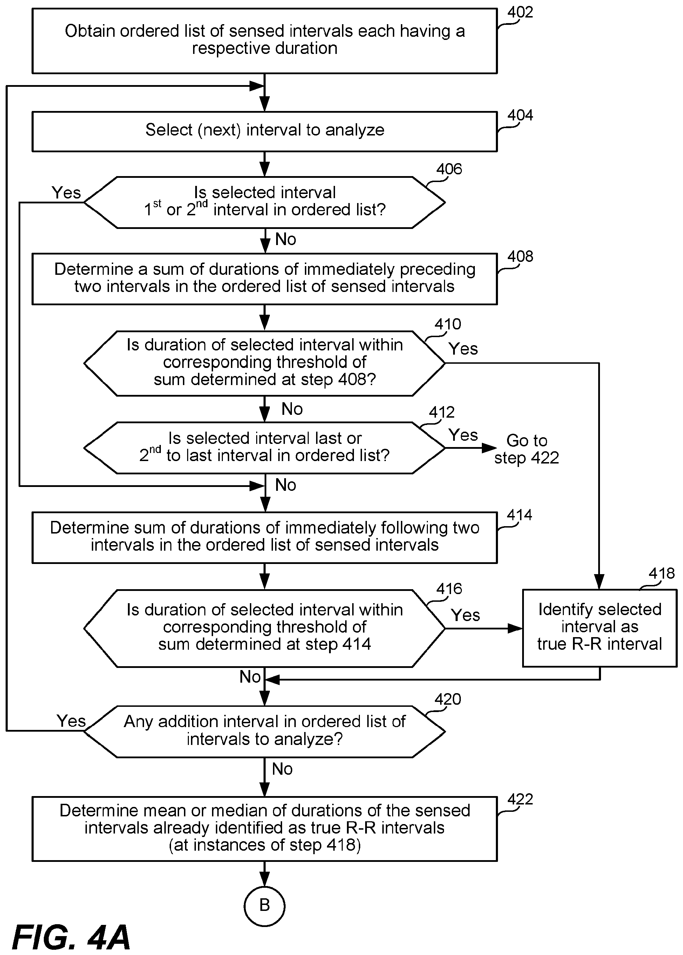

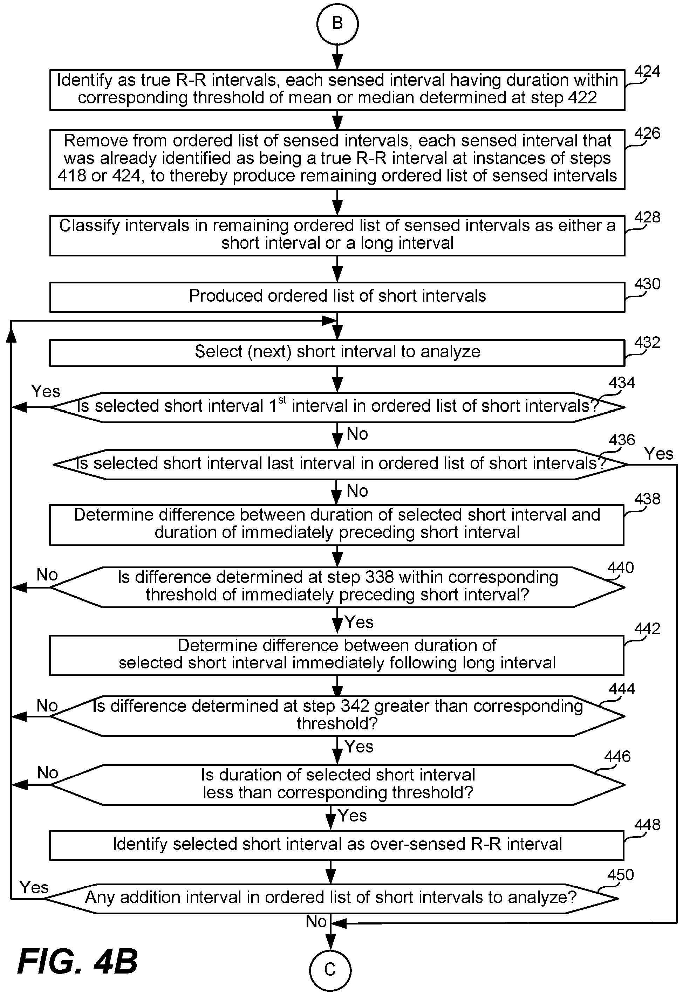

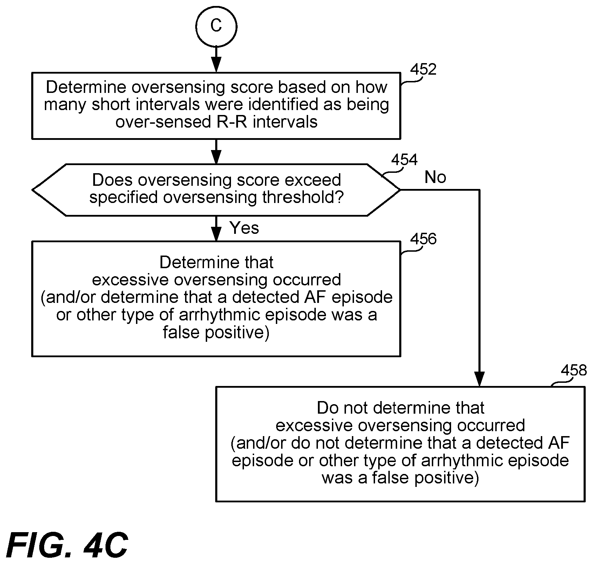

Additional and alternative details of the embodiments of the present technology, introduced above, are described below with reference to FIGS. 4A, 4B, and 4C, which can be referred to collectively as FIGS. 4A-4C, or even more succinctly as FIG. 4.

Referring to FIG. 4A, step 402 involves obtaining an ordered list of intervals that each have a respective duration. The ordered list of intervals, obtained at step 402, would preferably include only true R-R intervals. However, due to T-wave and/or P-wave oversensing, the ordered list of intervals obtained at step 402 would likely also include one or more over-sensed R-R intervals. In accordance with certain embodiments, the ordered list of sensed intervals is obtained by sensing or otherwise obtaining an EGM or ECG segment corresponding to a period of time preceding and leading up to an AF episode or other type of arrhythmic episode that was detected, identifying potential R-waves within the EGM or ECG segment, and determining intervals between consecutive ones of the potential R-waves to thereby produce the ordered list of sensed intervals. Such potential R-waves can be identified within the EGM or ECG segment by comparing the EGM segment, or samples thereof, to an R-wave sensing threshold, and identifying potential R-waves when the R-wave sensing threshold is reached or exceeded. Other variations are also possible and within the scope of the embodiments described herein.

Step 404 involves selecting an interval (from the ordered list of intervals obtained at step 402) to analyze. The first time step 404 is performed (for an ordered list of intervals), the first interval in the ordered list is selected. The second time step 404 is performed (for the ordered list of intervals), the second interval in the ordered list is selected, and so on.

At step 406 there is a determination of whether the interval (selected for analysis at step 404) is the first or second interval in the ordered list of intervals. If the selected interval is the first or second interval (i.e., if the answer to the determination at step 406 is Yes), then flow goes to step 414 (thereby skipping steps 408, 410, and 412). If the selected interval is not the first or second interval (i.e., if the answer to the determination at step 406 is No), then flow goes to step 408.

Step 408 involves determining a sum of the durations of the immediately preceding two intervals in the ordered list of sensed intervals. At step 410 there is a determination of whether the duration of the selected interval is within a corresponding threshold (e.g., within 10%) of the sum determined at step 408. If the duration of the selected interval is within the corresponding threshold (e.g., within 10%) of the sum determined at step 408 (i.e., if the answer to the determination at step 410 is Yes), then flow goes to step 418, and the selected interval is identified (aka classified) as a true R-R interval. If the duration of the selected interval is not within the corresponding threshold (e.g., within 10%) of the sum determined at step 408 (i.e., if the answer to the determination at step 410 is No), then flow goes to step 412.

At step 412 there is a determination of whether the selected interval is the last or second to last interval in the ordered list of sensed intervals. If the selected interval is the last or second to last interval (i.e., if the answer to the determination at step 412 is Yes), then flow goes to step 422 (thereby skipping steps 414, 416, and 420). If the selected interval is not the last or second to last interval (i.e., if the answer to the determination at step 412 is No), then flow goes to step 414.

Step 414 involves determining the sum of the durations of the immediately following two intervals in the ordered list of intervals. At step 416 there is a determination of whether the duration of the selected interval is within a corresponding threshold (e.g., within 10%) of the sum determined at step 414. If the duration of the selected interval is within a corresponding threshold (e.g., within 10%) of the sum determined at step 414 (i.e., if the answer to the determination at step 416 is Yes), then flow goes to step 418, and the selected interval is identified (aka classified) as a true R-R interval. If the duration of the selected interval is not within the corresponding threshold (e.g., within 10%) of the sum determined at step 414 (i.e., if the answer to the determination at step 416 is No), then flow goes to step 420.

At step 420 there is a determination of whether there is at least one additional interval (in the ordered list of sensed intervals) to analyze. If the answer to the determination at step 420 is Yes, then flow returns to step 404 where the next interval (in the ordered list of sensed intervals) is selected for analysis. If the answer to the determination at step 420 is No, then flow goes to step 422, which is discussed below.

Steps 404 through 420, summarized above, are used to remove true R-R intervals from the ordered list of intervals being analyzed, which is accomplished by removing all intervals that are within a corresponding threshold (e.g., 10%) of the sum of the immediately preceding two intervals within the list, or that are within a corresponding threshold (e.g., 10%) of the sum of the immediately following two intervals within the list, as such intervals are likely true R-R intervals with a neighboring over-sensed R-R interval. While it is likely that the same threshold (e.g., 10%) is used at both step 410 and step 416, that need not be the case. The threshold used at step 410 and/or 416 can be a percentage, such as 10%, or some higher or lower percentage. Alternatively, the threshold used at step 410 and/or 416 can be a specified value, e.g., 80 milliseconds (ms), or some higher or lower value. Other variations are also possible, and within the scope of the embodiments described herein.

Returning to the discussion of step 422, step 422 involves determining a mean or median of the durations of the sensed intervals already identified as true R-R intervals (at instances of steps 418). Accordingly, at step 422 a likely duration of true R-R intervals is determined. Flow then goes to step 424 in FIG. 4B. Step 424 involves identifying (aka classifying) as a true R-R interval, each sensed interval (in the remaining ordered list of sensed intervals) that has a duration that is within a corresponding threshold (e.g., 10%) of the mean or median determined at step 422. Thus, R-R intervals are also identified as true R-R intervals if they are within a corresponding threshold (e.g., 10%) of the mean or median of the previously identified true R-R intervals. The threshold used at step 424 can be a percentage, such as 10%, or some higher or lower percentage. Alternatively, the threshold used at step 424 can be a specified value, e.g., 80 milliseconds (ms), or some higher or lower value. Other variations are also possible, and within the scope of the embodiments described herein. It can be appreciated from the description herein that the terms "identified" and "classified", and the terms "identify" and "classify", are often referred to interchangeably herein.

Step 426 involves removing from the ordered list of sensed intervals, each sensed interval that was already identified as being a true R-R interval at an instance of step 418 or 424, to thereby produce a remaining ordered list of sensed intervals. Step 428 then involves classifying (aka identifying) intervals in the remaining ordered list of intervals as either a short interval or a long interval. For an example, an interval (in the remaining ordered list of sensed intervals) can be classified as a short interval when a duration of the interval is shorter than an immediately following interval in the remaining ordered list of sensed intervals; and an interval (in the remaining ordered list of sensed intervals) can be classified as a long interval when a duration of the interval is longer than an immediately following interval in the remaining ordered list of sensed intervals. Other ways of classifying intervals as short or long intervals are also possible and within the scope of the embodiments described herein. For example, rather than (or in addition to) comparing a duration of an interval to the duration of the following interval, the duration of the interval can be compared to the duration of the preceding interval. In certain embodiments, the first and/or last interval (in the remaining ordered list of sensed intervals) is removed or ignored.

Step 430 involves producing an ordered list of the short intervals. This can be accomplished, for example, by removing all the long intervals (identified at step 428) from the remaining ordered list of sensed intervals (produced at step 426). This can alternatively be accomplished by creating a new ordered list that just includes the short intervals identified at step 428. Other variations are also possible and within the scope of the embodiments described herein.

Step 432 involves selecting an interval (from the ordered list of short intervals produced at step 430) to analyze. The first time step 432 is performed (for an ordered list of short intervals), the first interval in the ordered list is selected. The second time step 432 is performed (for the ordered list of short intervals), the second interval in the ordered list is selected, and so on.

At step 434 there is a determination of whether the interval (selected for analysis at step 432) is the first interval in the ordered list of short intervals. If the selected interval is the first interval (i.e., if the answer to the determination at step 434 is Yes), then flow goes back to step 432 and the next interval is selected for analysis. If the selected interval is not the first interval (i.e., if the answer to the determination at step 434 is No), then flow goes to step 436.

At step 436 there is a determination of whether the interval (selected for analysis at step 432) is the last interval in the ordered list of short intervals. If the selected interval is the last interval (i.e., if the answer to the determination at step 436 is Yes), then flow skips to step 452. If the selected interval is not the last interval (i.e., if the answer to the determination at step 436 is No), then flow goes to step 438.

Step 438 involves determining a difference between the duration of the selected short interval and the duration of the immediately preceding short interval. At step 440 there is a determination of whether the difference determined at step 438 is within a corresponding threshold (e.g., 10%) of the immediately preceding short interval. If the answer to the determination at step 440 is No, then flow returns to step 432 where the next short interval to analyze is selected. If the answer to the determination at step 440 is Yes, then flow goes to step 442. Steps 438 and 440 are used to test the short interval variability criteria, because the "short" intervals that truly correspond to oversensing (i.e., that are over-sensed R-R intervals) should have low interval-interval variability, e.g., quantified by a difference from the previous "short" interval of less than 10% or some other specified threshold.

Step 442 involves determining a difference between the duration of the selected short interval and the duration of the immediately following long interval. At step 444 there is a determination of whether the difference determined at step 442 is greater than a corresponding threshold (e.g., 5%) of the immediately following long interval. If the answer to the determination at step 444 is No, then flow returns to step 432 where the next short interval to analyze is selected. If the answer to the determination at step 444 is Yes, then flow goes to step 446. The threshold used at step 444 can be a percentage, such as 5%, or some higher or lower percentage. Alternatively, the threshold used at step 446 can be a specified value, e.g., 20 milliseconds (ms), or some higher or lower value. Other variations are also possible, and within the scope of the embodiments described herein. Steps 442 and 444 are used to test the short-long interval discrepancy, because a short interval that truly correspond to oversensing should differ from the next long interval by more than 5% or some other specified threshold.

Step 446 involves determining whether the duration of the selected short interval is less than a corresponding threshold, e.g., 444 ms (i.e., greater than 135 bpm). If the answer to the determination at step 446 is No, then flow returns to step 432 where the next short interval to analyze is selected. If the answer to the determination at step 446 is Yes, then flow goes to step 448. Step 446 is used to test the short interval duration, which handles the possibility that all of the above criteria could be satisfied by slow, yet variable, heart rates. During true oversensing, a short interval should correspond to a very fast heart rate, which is tested for at step 446.

At step 448 the short interval (for which the answers to the determinations at step 440, 444, and 446 were all Yes) is identified (aka classified) as an over-sensed R-R interval. This is because the interval satisfied the short interval variability criteria, the short-long interval discrepancy criteria, and the short interval duration criteria, which were discussed above.

At step 450 there is a determination of whether there is at least one additional short interval (in the ordered list of short intervals) to analyze. If the answer to the determination at step 450 is Yes, then flow returns to step 432 where the next short interval (in the ordered list of short intervals) is selected for analysis. If the answer to the determination at step 450 is No, then flow goes to step 452 in FIG. 4C, which is discussed below.

Referring to FIG. 4C, step 452 involves determining an oversensing score based on how many short intervals were identified as being over-sensed R-R intervals (at instances of step 448). In accordance with certain embodiments, the oversensing score is determined by determining what percentage of all of the sensed intervals classified as a short interval were identified as being an over-sensed R-R interval. Another way to calculate the oversensing score is using the equation Oversensing Score (OS) = 2 x [the total number of sensed intervals classified as a short interval that were identified as being an over-sensed R-R interval] / [total number of all intervals]. The factor of "2" in this equation accounts for the fact that each "short" interval also has a paired "long" interval, so it should count as 2 of the total intervals when calculating a percent. In other words, both intervals associated with (i.e., before and after) an oversensed P- or T-wave are counted. Other variations are also possible and within the scope of the embodiments described herein.

At step 454 there is a determination of whether the oversensing score (determined at step 452) exceeds a corresponding threshold (e.g., 50%). If the oversensing score exceeds the corresponding threshold (e.g., if more than 50% of the short intervals were classified as being over-sensed intervals), then flow goes to step 456. At step 456 there is a determination or conclusion that excessive oversensing had occurred. Such a determination or conclusion can be used to determine that a detected AF episode (or other type of arrhythmic episode) that was detected based on the sensed intervals was a false positive. If the oversensing score does not exceed the corresponding threshold (e.g., if less than 50% of the short intervals were classified as being over-sensed intervals), then flow goes to step 458, and there is no conclusion or determination that excessive oversensing occurred. In accordance with certain embodiments, different corresponding thresholds can be used for different types of tachycardias. For example, the corresponding threshold to which an oversensing score is compared when determining whether the detection of an AF episode was a false positive detection could be 50%, and the corresponding threshold to which an oversensing score is compared when determining whether the detection of a VT episode was a false positive detection could be 70%.

In accordance with certain embodiments, an IMD may perform the method described above with reference to FIG. 4 in response to an AF episode (or some other type of arrhythmic episode) being detected. The detection of an AF episode can also be referred to as an AF trigger. Such an IMD may be configured to transmit, to an external device that is communicatively coupled to a patient care network, data corresponding to an AF episode (or other type of arrhythmic episode) that is detected by the IMD. In certain such embodiments, the IMD does not (is prevented from) transmitting (to the external device that is communicatively coupled to the patient care network) data corresponding to an AF episode (or other type of arrhythmic episode) that is detected by the IMD, but is thereafter determined by the IMD as being a false positive detection.

In accordance with certain embodiments, the medical device (e.g., IMD) that performs the method described above with reference to FIG. 4 may monitor the HR of a patient based on intervals identified from a segment of an EGM or ECG, and the medical device can determine based on the results of the method whether a monitored HR is inaccurate due to oversensing and thus should be ignored or recalculated. For an example, if an oversensing score exceeds a corresponding threshold, the medical device can conclude that a HR that was determined based on sensed interval is inaccurate and should not be used, or should be recalculated.

In accordance with certain embodiments, after identifying true R-R intervals (e.g., at instances of steps 418 and/or 424) and identifying (aka classifying) individual over-sensed R-R intervals short or long intervals, pairs of over-sensed R-R intervals can be combined to identify further true R-R intervals. For example, a short interval (e.g., R-T interval) and a following long interval (e.g., T-R interval) can be summed to produce a calculated true R-R interval. For another example, adjacent R-P and P-R intervals can be summed to produce a calculated true R-R interval. A corrected ordered list of sensed intervals can then be produced that includes the true R-R intervals (e.g., identified at instances of steps 418 and/or 424) and the further true R-R intervals identified by summing appropriate pairs of over-sensed R-R intervals. HR monitoring and/or monitoring for an arrhythmic episode can then be based on the corrected ordered list of sensed intervals.

An implementation of an embodiment of the present technology described above was tested to determine whether and to what extend the present technology can be used to reduce the reporting of false positive AF detections. EGM segments corresponding to a total of 5,989 detected AF episodes (that were detected by Confirm Rx^{™} ICMs) were manually adjudicated as either "true" or "false" positive AF detections. Among other characteristics, the heart rate (HR), heart rate variability (HRV), lack of heart rate patterns, lack of visible P-waves, variable R-wave morphology, and P:R ratio were used for the manual adjudication. Note that the manual adjudication relied on both the R-R intervals and the EGM signal morphology, and required thousands of human-hours to perform. Using only the R-R interval values in the 30 seconds preceding each AF detection trigger, an embodiment of the present technology described above was used to automatically calculate the "oversensing score" of all episodes in under 10 seconds. Of the 5,989 episodes, 1469 episodes (24.5%) were adjudicated as true AF episodes (i.e., as true positives), and the remaining 4,520 episodes (75.5%) were adjudicated as non-AF episodes (i.e., as false positives). Of the 4520 manually adjudicated non-AF episodes (i.e., false positive AF detections), 303 (6.7%) were flagged by the present technology as oversensing (and thus, as being false positives) because the oversensing score was greater than a 50% threshold. This corresponds to a potential 6.7% reduction in false positives using an interval-based embodiment of the present technology. Note that false positive AF episodes can be the result of many other phenomena besides oversensing, such as, but not limited to, R-wave undersensing, noise, ventricular tachycardia, PVCs, or atrial flutter. Overall, 305 detected AF episodes were flagged as false positives using an embodiment of the present technology. Of those, 303 detected AF episodes (99.3%) were indeed manually adjudicated as non-AF episodes. Beneficially, only 2 of the 1469 detected AF episodes (0.1%) were incorrectly flagged as false positives. Embodiments of the present technology can be used together with other types of technology that can be used to identify further false positives, or more generally, excessive oversensing.

The specific thresholds used to test the performance of embodiments of the present technology (e.g. 10% interval stability, 5% interval discrepancy, 135 bpm heart rate lower limit, 50% oversensing score threshold) and mentioned above can be more systematically optimized for a broader patient population, or for individual patients. Accordingly, embodiments of the present technology described herein should not be limited to use with the exemplary thresholds described herein.

While embodiments of the present technology described herein have often been described as being used to determine whether a detected AF episode was a false positive detection, such embodiments can alternatively or additionally be used to determine whether detections of other types of supraventricular tachycardia episodes, and/or detections of ventricular tachycardia (VT) episodes, are false positive detections.

The specific window or ordered list of sensed intervals that is analyzed to determine whether a detected tachycardia episode was a false positive detection may depend on the specific type of tachycardia that was detected. For example, where an AF episode was detected, the ordered list of sensed intervals that is analyzed can include sensed intervals corresponding to a period of time (e.g., 30 seconds) preceding and leading up to the detected AF episode. By contrast, where a VT episode was detected, the ordered list of sensed intervals that is analyzed can be those sensed intervals spanning and inclusive of the first and last tachy-classified sensed intervals. Other variations are also possible and within the scope of the embodiments described herein.

Embodiments of the present technology described herein can be used with various types of IMDs, including, but not limited to, an insertable cardiac monitor (ICM), a cardiac pacemaker to which one or more leads is/are attached, a leadless cardiac pacemaker (LCP), or an implantable cardioverter defibrillator (ICD). Such an ICD can be a transvascular ICD, or a nonvascular ICD, wherein the nonvascular ICD can be a subcutaneous (SubQ) ICD. Where embodiments of the present technology are implemented by an ICM, such embodiments can be used, e.g., to reduce the number of false positive AF detections that are transmitted from the ICM to a patient care network for clinician review. This is beneficially because false positive AF detections are highly undesirable, as the burden of sorting through large numbers of clinically irrelevant episodes of AF can be time consuming and costly. Where embodiments of the present technology are used by an ICD, or by an IMD in communication with an ICD, such embodiments can reduce how often anti-tachycardia pacing (ATP) and/or defibrillation shocks are delivered in response to false positive tachycardia detections. This is beneficial because defibrillation shocks are typically painful, and delivering such shocks when patient is awake in response to false positive tachycardia detections subjects the patient to unnecessary painful or uncomfortable shocks and may prematurely deplete the energy stored in a battery.

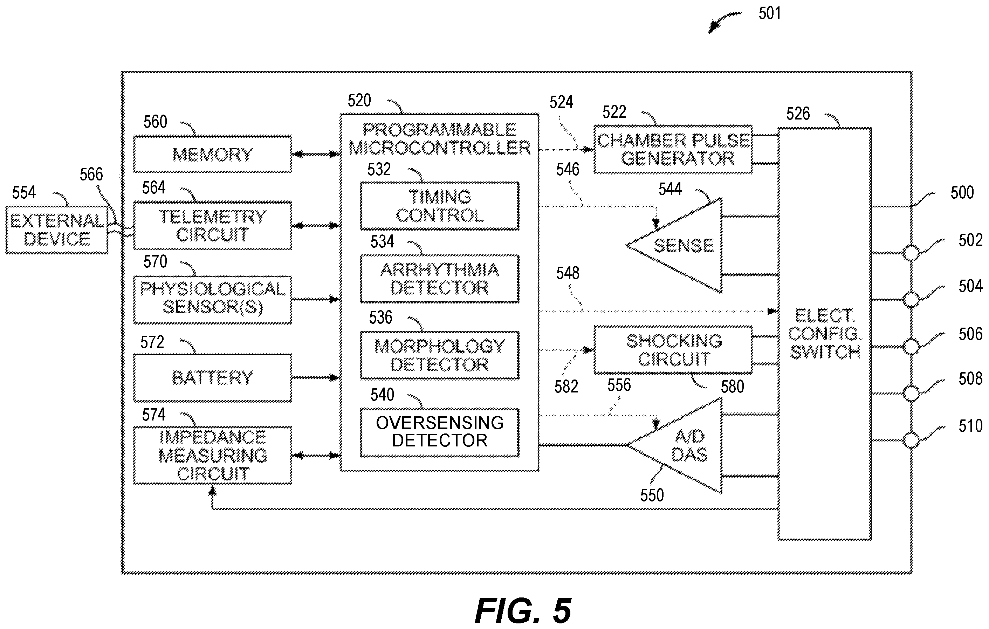

FIG. 5 shows a block diagram of one embodiment of an IMD that is implanted into a patient in accordance with a certain embodiment of the present technology. The IMD 501 may be implemented as a full-function biventricular pacemaker, equipped with both atrial and ventricular sensing and pacing circuitry for four chamber sensing and stimulation therapy (including both pacing and shock treatment). Optionally, the IMD 501 may provide full-function cardiac resynchronization therapy. Alternatively, the IMD 501 may be implemented with a reduced set of functions and components. For instance, the IMD may be implemented without pacing, e.g., if the IMD is an ICM. The IMD 501 can be coupled to one or more leads for single chamber or multi-chamber pacing and/or sensing. Alternatively, the IMD 501 can be an LCP that includes electrodes located on or very close to a housing 500 of the IMD 501.

The IMD 501 has a housing 500 to hold the electronic/computing components. The housing 500 (which is often referred to as the "can", "case", "encasing", or "case electrode") may be programmably selected to act as the return electrode for certain stimulus modes. The housing 500 may further include a connector (not shown) with a plurality of terminals 502, 504, 506, 508, and 510. The terminals may be connected to electrodes that are located in various locations on the housing 500 or to electrodes located on leads. The IMD 501 includes a programmable microcontroller 520 that controls various operations of the IMD 501, including cardiac monitoring and/or stimulation therapy. The microcontroller 520 includes a microprocessor (or equivalent control circuitry), RAM and/or ROM memory, logic and timing circuitry, state machine circuitry, and I/O circuitry.

The IMD 501 further includes a pulse generator 522 that generates stimulation pulses and communication pulses for delivery by one or more electrodes coupled thereto. The pulse generator 522 is controlled by the microcontroller 520 via a control signal 524. The pulse generator 522 may be coupled to the select electrode(s) via an electrode configuration switch 526, which includes multiple switches for connecting the desired electrodes to the appropriate I/O circuits, thereby facilitating electrode programmability. The switch 526 is controlled by a control signal 528 from microcontroller 520.

In the embodiment of FIG. 5, a single pulse generator 522 is illustrated. Optionally, the IMD may include multiple pulse generators, similar to the pulse generator 522, where each pulse generator is coupled to one or more electrodes and controlled by the microcontroller 520 to deliver select stimulus pulse(s) to the corresponding one or more electrodes.

The microcontroller 520 is illustrated as including timing control circuitry 532 to control the timing of the stimulation pulses (e.g., pacing rate, atrio-ventricular (AV) delay, atrial interconduction (A-A) delay, or ventricular interconduction (V-V) delay, etc.). The timing control circuitry 532 may also be used for the timing of refractory periods, blanking intervals, noise detection windows, evoked response windows, alert intervals, marker channel timing, and so on. The microcontroller 520 also has an arrhythmia detector 534 for detecting arrhythmia conditions and a morphology detector 536. Although not shown, the microcontroller 520 may further include other dedicated circuitry and/or firmware/software components that assist in monitoring various conditions of the patient's heart and managing pacing therapies. The microcontroller 520 is also shown as including an oversensing detector 540, which can be used to perform the embodiments of the present technology described above with reference to FIGS. 1-4. The oversensing detector 540 can more generally be implemented using hardware, software, firmware, and/or combinations thereof. The microcontroller can include a processor. The microcontroller, and/or the processor thereof, can be used to perform the methods of the present technology described herein.

The IMD 501 can be further equipped with a communication modem (modulator/demodulator) to enable wireless communication with the remote slave pacing unit. The modem may include one or more transmitters and two or more receivers. In one implementation, the modem may use low or high frequency modulation. As one example, modem may transmit implant-to-implant (i2i) messages and other signals through conductive communication between a pair of electrodes. Such a modem may be implemented in hardware as part of the microcontroller 520, or as software/firmware instructions programmed into and executed by the microcontroller 520. Alternatively, the modem may reside separately from the microcontroller as a standalone component.

The IMD 501 includes a sensing circuit 544 selectively coupled to one or more electrodes, that perform sensing operations, through the switch 526 to detect the presence of cardiac activity in the right chambers of the heart. The sensing circuit 544 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. The switch 526 determines the sensing polarity of the cardiac signal by selectively closing the appropriate switches. In this way, the clinician may program the sensing polarity independent of the stimulation polarity.

The output of the sensing circuit 544 is connected to the microcontroller 520 which, in turn, triggers or inhibits the pulse generator 522 in response to the presence or absence of cardiac activity. The sensing circuit 544 receives a control signal 546 from the microcontroller 520 for purposes of controlling the gain, threshold, polarization charge removal circuitry (not shown), and the timing of any blocking circuitry (not shown) coupled to the inputs of the sensing circuitry.

In the embodiment of FIG. 5, a single sensing circuit 544 is illustrated. Optionally, the IMD may include multiple sensing circuits, similar to the sensing circuit 544, where each sensing circuit is coupled to one or more electrodes and controlled by the microcontroller 520 to sense electrical activity detected at the corresponding one or more electrodes. The sensing circuit 544 may operate in a unipolar sensing configuration or in a bipolar sensing configuration.

The IMD 501 further includes an analog-to-digital (A/D) data acquisition system (DAS) 550 coupled to one or more electrodes via the switch 526 to sample cardiac signals across any pair of desired electrodes. Data acquisition system 550 is configured to acquire intracardiac electrogram signals, convert the raw analog data into digital data, and store the digital data for later processing and/or telemetric transmission to an external device 554 (e.g., a programmer, local transceiver, or a diagnostic system analyzer). Data acquisition system 550 is controlled by a control signal 556 from the microcontroller 520.

The microcontroller 520 is coupled to a memory 560 by a suitable data/address bus. The programmable operating parameters used by the microcontroller 520 are stored in memory 560 and used to customize the operation of the IMD 501 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy.

The operating parameters of the IMD 501 may be non-invasively programmed into memory 560 through a telemetry circuit 564 in telemetric communication via a communication link 566 with an external device 554. The telemetry circuit 564 allows intracardiac electrograms and status information relating to the operation of the IMD 501 (as contained in the microcontroller 520 or memory 560) to be sent to the external device 554 through the communication link 566.

The IMD 501 can further include magnet detection circuitry (not shown), coupled to the microcontroller 520, to detect when a magnet is placed over the unit. A magnet may be used by a clinician to perform various test functions of IMD 501 and/or to signal the microcontroller 520 that the external device 554 is in place to receive or transmit data to the microcontroller 520 through the telemetry circuit 564.

The IMD 501 can further include one or more physiological sensors 570. Such sensors are commonly referred to as "rate-responsive" sensors because they are typically used to adjust pacing stimulation rates according to the exercise state of the patient. However, the physiological sensor(s) 570 may further be used to detect changes in cardiac output, changes in the physiological condition of the heart, or diurnal changes in activity (e.g., detecting sleep and wake states). Signals generated by the physiological sensor(s) 570 are passed to the microcontroller 520 for analysis. The microcontroller 520 responds by adjusting the various pacing parameters (such as rate, AV Delay, V-V Delay, etc.) at which the atrial and ventricular pacing pulses are administered. While shown as being included within the IMD 501, one or more physiological sensor(s) 570 may be external to the IMD 501, yet still be implanted within or carried by the patient. Examples of physiologic sensors include sensors that, for example, sense respiration rate, pH of blood, ventricular gradient, activity, position/posture, minute ventilation (MV), and so forth.

A battery 572 provides operating power to all of the components in the IMD 501. The battery 572 is preferably capable of operating at low current drains for long periods of time, and is capable of providing high-current pulses (for capacitor charging) when the patient requires a shock pulse (e.g., in excess of 2 A, at voltages above 2 V, for periods of 10 seconds or more). The battery 572 also desirably has a predictable discharge characteristic so that elective replacement time can be detected. As one example, the IMD 501 employs lithium/silver vanadium oxide batteries.

The IMD 501 further includes an impedance measuring circuit 574, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. The impedance measuring circuit 574 is coupled to the switch 526 so that any desired electrode may be used. In this embodiment the IMD 501 further includes a shocking circuit 580 coupled to the microcontroller 520 by a data/address bus 582.

The embodiments of the present technology described above were primarily described as being used with an implantable medical device or system that monitors HR and/or for one or more types of arrhythmic episodes based on sensed intervals, which as noted above can be, e.g., true R-R intervals or over-sensed R-R intervals. Such embodiments of the present technology can alternatively be used with a non-implantable device or system (aka an external device or system) that includes at least two electrodes in contact with a person's skin and is used to monitor HR and/or for one or more types of arrhythmic episodes based on sensed intervals. More specifically, such embodiments can alternatively be used with or be implemented by a user wearable device, such as a wrist worn device, or a user wearable device designed to be worn on one or more other portions of a person's body besides a wrist, e.g., on an ankle, an upper arm, or a chest, but not limited thereto. Such a user wearable device can include electrodes that are configured to contact a person's skin, sensing circuity coupled to the electrodes and configured to obtain a signal indicative of electrical activity of a patient's heart, and at least one of a processor or controller that is configured to perform one or more of the algorithms described above. Such a user wearable device (or more generally an external device or system) can monitor for AF and/or other types of arrythmia(s) and determine when there is a false positive detection. Additionally, or alternatively, such a user wearable device (or more generally an external device or system) can monitor a person's HR and determine when measures of HR are likely inaccurate due to oversensing. A user wearable device can both obtain a signal indicative of electrical activity of a patient's heart and monitor a person's HR and/or for arrythmia(s) based on intervals obtained from the obtained signal. Alternatively, a user wearable device can be communicatively coupled to another external device, such as a smartphone or tablet computer, and the other external device can obtain the signal from the user wearable device and monitor a person's HR and/or for arrythmia(s) based on intervals. The user wearable device or other external device or system can determine when there may be a false positive and/or when a measured HR may be inaccurate due to oversensing. Other implementations of such an external device or system are also possible and within the scope of the embodiments described herein.

## Claims

1. A device (501), comprising:
one or more electrodes (500, 502, 504, 506, 508, 510);
a sensing circuit (544) coupled to the one or more electrodes (500, 502, 504, 506, 508, 510) and configured to obtain a signal indicative of electrical activity of a patient's heart; and
at least one of a processor or controller (520) configured to:
obtain an ordered list of sensed intervals, ordered in the order the intervals have been sensed, wherein the sensed intervals are determined between consecutive ones of potential R-waves in the signal indicative of the electrical activity of the patient's heart, wherein each of the sensed intervals has a respective duration, wherein the ordered list of sensed intervals include true R-R intervals and one or more over-sensed R-R intervals due to T-wave and/or P-wave oversensing, wherein a true R-R interval is an interval between consecutive actual R-waves, and wherein an over-sensed R-R interval is a P-R interval between an over-sensed P-wave and a consecutive actual R-wave, a R-P interval between an actual R-wave and a consecutive over-sensed P-wave, a T-R interval between an over-sensed T-wave and a consecutive actual R-wave, or a R-T interval between an actual R-wave and a consecutive over-sensed T-wave;
identify actual R-R intervals as true R-R intervals within the ordered list of sensed intervals;
remove from the ordered list of sensed intervals each of the sensed intervals that was identified as being a true R-R interval, to thereby produce a remaining ordered list of sensed intervals;
classify individual intervals, in the remaining ordered list of sensed intervals, as either a short interval when a duration of the interval is shorter than the duration of an immediately following interval and/or the duration of an immediately preceding interval in the remaining ordered list of sensed intervals, or a long interval when a duration of the interval is longer than the duration of an immediately following interval and/or the duration of an immediately preceding interval in the remaining ordered list of sensed intervals; and
identify sensed intervals in the remaining ordered list of sensed intervals as over-sensed R-R intervals based on an analysis of at least the short intervals in the remaining ordered list of sensed intervals,
wherein in order to identify true R-R intervals within the ordered list of sensed intervals the at least one of a processor or controller (520) is/are configured to:
identify (418) as a said true R-R interval, each said sensed interval having a duration that is within a first specified threshold of either (i) a sum of the durations of an immediately preceding two intervals in the ordered list of sensed intervals (408), or (ii) a sum of the durations of an immediately following two intervals in the ordered list of sensed intervals (414); and
identify (424) as a said true R-R interval, each said sensed interval having a duration that is within a second specified threshold of a mean or median of the durations of the sensed intervals already identified as true R-R intervals, wherein the second specified threshold may or may not be the same as the first specified threshold; and
wherein in order to identify a said sensed interval, that has been classified as a said short interval in the remaining ordered list of sensed intervals, as an over-sensed R-R interval, the at least one of a processor or controller (520) is/are configured to identify the sensed interval as an over-sensed R-R interval when all of the following are true:
a difference between a duration of the sensed interval that was classified as a said short interval and a duration of an immediately preceding sensed interval that was classified as a said short interval is within a third specified threshold (438, 440);
a difference between the duration of the sensed interval that was classified as a said short interval and a duration of an immediately following sensed interval that was classified as a said long interval is greater than a fourth specified threshold (442, 444); and
the duration of the sensed interval that was classified as a said short interval is less than a fifth specified threshold (446).

2. The device of claim 1, wherein for each sensed interval classified as a said long interval, in the remaining ordered list of sensed intervals, the at least one of a processor or controller (520) is/are configured to identify the sensed interval as an over-sensed R-R interval if an immediately preceding or following sensed interval, in the remaining ordered list of sensed intervals, is classified as a said short interval and has been identified as an over-sensed R-R interval.

3. The device of any one of claims 1 through 2, wherein the at least one of a processor or controller (520) is/are further configured to:
determine, based on which individual intervals are identified as over-sensed R-R intervals, whether a monitored heart rate or arrhythmic episode detection is inaccurate due to oversensing and thus should be ignored or recalculated.

4. The device of claim 3, wherein in order to determine, based on which individual intervals are identified as over-sensed R-R intervals, whether a monitored heart rate or arrhythmic episode detection is inaccurate due to oversensing and thus should be ignored or recalculated, the at least one of a processor or controller (520) is/are configured to:
determine (452) an oversensing score based on how many of the sensed intervals classified as a said short interval were identified as being an over-sensed R-R interval; and
determine (454-458), based on the oversensing score, whether a monitored heart rate or arrhythmic episode detection is inaccurate due to oversensing and thus should be ignored or recalculated.

5. The device of any one of claims 1 through 4, wherein the at least one of a processor or controller (520) is/are further configured to:
combine pairs of over-sensed R-R intervals to identify further true R-R intervals;
produce a corrected ordered list of sensed intervals including true R-R intervals, at least some of which are identified by combining pairs of over-sensed R-R intervals; and
monitor heart rate and/or for an arrhythmic episode based on the corrected ordered list of sensed intervals.

6. The device of any one of claims 1 through 5, wherein the at least one of a processor or controller (520) is/are configured to combine said pairs of over-sensed R-R intervals, produce the corrected ordered list of sensed intervals, and monitor heart rate and/or for an arrhythmic episode based on the corrected ordered list of sensed intervals, in response to there being a determination that a monitored heart rate or arrhythmic episode detection is inaccurate due to oversensing and thus should be ignored or recalculated.

7. The device of any one of claims 1 through 6, further comprising a transceiver (564) configured to wireless communicate with an external device (554), and wherein the at least one of a processor or controller (520) is/are further configured to interact with the transceiver (564) to:
cause data corresponding to an atrial fibrillation (AF) episode or other type of arrhythmic episode that is detected by the device (501) to be selectively transmitted to the external device (554); and
prevent data corresponding to an AF episode or other type of arrhythmic episode that is detected by the device (501), but is thereafter determined by the device (501) as being a false positive detection, from being transmitted to the external device (554).

8. The device of any one of claims 1 through 7, wherein the device (501) comprises one of the following:
a user wearable device;
an insertable cardiac monitor (ICM);
a cardiac pacemaker to which one or more leads is/are attached;
a leadless cardiac pacemaker (LCP); or
an implantable cardioverter defibrillator (ICD).

9. A method performed by a device (501) or system that monitors a heart rate of a patient's heart, the method comprising:
obtaining (402) an ordered list of sensed intervals, ordered in the order the intervals have been sensed, wherein the sensed intervals are determined between consecutive ones of potential R waves in a signal indicative of the electrical activity of the patient's heart, wherein each of the sensed intervals has a respective duration, wherein the ordered list of sensed intervals include true R-R intervals and one or more over-sensed R-R intervals due to T-wave and/or P-wave oversensing, wherein a true R-R interval is an interval between consecutive actual R-waves, and wherein an over-sensed R-R interval is a P-R interval between an over-sensed P-wave and a consecutive actual R-wave, a R-P interval between an actual R-wave and a consecutive over-sensed P-wave, a T-R interval between an over-sensed T-wave and a consecutive actual R-wave, or a R-T interval between an actual R-wave and a consecutive over-sensed T-wave;
identifying (402-424) an actual R-R interval as a true R-R interval within the ordered list of sensed intervals;
removing (426), from the ordered list of sensed intervals, each of the sensed intervals that was identified as being a true R-R interval, to thereby produce a remaining ordered list of sensed intervals;
classifying (428) individual intervals in the remaining ordered list of sensed Intervals as either a short interval when a duration of the interval is shorter than the duration of an immediately following interval and/or the duration of an immediately preceding interval in the remaining ordered list of sensed intervals, or a long interval when a duration of the interval is longer than the duration of an immediately following interval and/or the duration of an immediately preceding interval in the remaining ordered list of sensed intervals; and
identifying (430-450) sensed intervals in the remaining ordered list of sensed intervals as one or more over-sensed R-R intervals based on an analysis of at least the short intervals in the remaining ordered list of sensed intervals,
wherein the identifying true R-R intervals (404-424) within the ordered list of sensed intervals comprises:
identifying (408, 410, 414, 416, 418) as a said true R-R interval, each said sensed interval having a duration that is within a first specified threshold of either (i) a sum of the durations of an immediately preceding two intervals in the ordered list of sensed intervals, or (ii) a sum of the durations of an immediately following two intervals in the ordered list of sensed intervals; and
identifying (422, 424) as a said true R-R interval, each said sensed interval having a duration that is within a second specified threshold of a mean or median of the durations of the sensed intervals already identified as true R-R intervals, wherein the second specified threshold may or may not be the same as the first specified threshold; and
wherein the identifying (430, 450) one or more over-sensed R-R intervals comprises, for at least one said sensed interval classified as a said short interval in the remaining ordered list of sensed intervals, identifying the sensed interval as an over-sensed R-R interval when all of the following are true:
(438, 440) a difference between a duration of the sensed interval that was classified as a said short interval and a duration of an immediately preceding sensed interval that was classified as a said short interval is within a third specified threshold;
(442, 444) a difference between the duration of the sensed interval that was classified as a said short interval and a duration of an immediately following sensed interval that was classified as a said long interval is greater than a fourth specified threshold; and
(446) the duration of the sensed interval that was classified as a said short interval is less than a fifth specified threshold.

10. The method of claim 9, wherein the identifying (430-450) one or more over-sensed R-R intervals based on results of the classifying each of the intervals in the remaining ordered list of sensed intervals as either a said short interval or a said long interval, further comprises:
for each sensed interval classified as a said long interval, in the remaining ordered list of sensed intervals, identifying the sensed interval as an over-sensed R-R interval if an immediately preceding or following sensed interval classified as a said short interval has been identified as an over-sensed R-R interval.

11. The method of any one of claims 9 through 10, further comprising:
determining (452) an oversensing score based on how many of the sensed intervals classified as a said short interval were identified as being an over-sensed R-R interval; and
determining (454-456) that a detected AF episode or other type of arrhythmic episode was a false positive detection when the oversensing score exceeds a specified oversensing threshold.

12. The method of claim 11, wherein:
the specified oversensing threshold comprises a specified percentage threshold;
the determining (452) the oversensing score comprises determining what percentage of all of the sensed intervals classified as a said short interval were identified as being an over-sensed R-R interval; and
the determining (454-456) that a detected AF episode or other type of arrhythmic episode was a false positive detection occurs when the determined percentage exceeds the specified percentage threshold.

## Patentansprüche

1. Vorrichtung (501), umfassend:
eine oder mehrere Elektroden (500, 502, 504, 506, 508, 510);
eine Erfassungsschaltung (544), die an die eine oder die mehreren Elektroden (500, 502, 504, 506, 508, 510) gekoppelt und dazu konfiguriert ist, ein Signal zu erhalten, das eine elektrische Aktivität eines Herzens eines Patienten angibt; und
mindestens eines von einem Prozessor oder einer Steuerung (520), der/die zu Folgendem konfiguriert ist:
Erhalten einer geordneten Liste von erfassten Intervallen, geordnet in der Reihenfolge, in der die Intervalle erfasst wurden, wobei die erfassten Intervalle zwischen aufeinanderfolgenden von potenziellen R-Wellen in dem Signal, das die elektrische Aktivität des Herzens eines Patienten angibt, bestimmt werden, wobei jedes der erfassten Intervalle eine jeweilige Dauer aufweist, wobei die geordnete Liste von erfassten Intervallen echte R-R-Intervalle und ein oder mehrere übererfasste R-R-Intervalle aufgrund von T-Wellen- und/oder P-Wellenübererfassung beinhaltet, wobei ein echtes R-R-Intervall ein Intervall zwischen aufeinanderfolgenden tatsächlichen R-Wellen ist und wobei ein übererfasstes R-R-Intervall ein P-R-Intervall zwischen einer übererfassten P-Welle und einer darauffolgenden tatsächlichen R-Welle, ein R-P-Intervall zwischen einer tatsächlichen R-Welle und einer darauffolgenden übererfassten P-Welle, ein T-R-Intervall zwischen einer übererfassten T-Welle und einer darauffolgenden tatsächlichen R-Welle oder ein R-T-Intervall zwischen einer tatsächlichen R-Welle und einer darauffolgenden übererfassten T-Welle ist;
Identifizieren tatsächlicher R-R-Intervalle als echte R-R-Intervalle innerhalb der geordneten Liste von erfassten Intervallen;
Entfernen, aus der geordneten Liste erfasster Intervalle, eines jeden der erfassten Intervalle, das als ein echtes R-R-Intervall identifiziert wurde, um dadurch eine verbleibende geordnete Liste von erfassten Intervallen zu erzeugen;
Klassifizieren individueller Intervalle in der verbleibenden geordneten Liste von erfassten Intervallen entweder als ein kurzes Intervall, wenn eine Dauer des Intervalls kürzer als die Dauer eines unmittelbar folgenden Intervalls und/oder die Dauer eines unmittelbar vorhergehenden Intervalls in der verbleibenden geordneten Liste von erfassten Intervallen ist, oder als ein langes Intervall, wenn eine Dauer des Intervalls länger als die Dauer eines unmittelbar folgenden Intervalls und/oder die Dauer eines unmittelbar vorhergehenden Intervalls in der verbleibenden geordneten Liste von erfassten Intervallen ist; und
Identifizieren erfasster Intervalle in der verbleibenden geordneten Liste von erfassten Intervallen als übererfasste R-R-Intervalle basierend auf einer Analyse mindestens der kurzen Intervalle in der verbleibenden geordneten Liste von erfassten Intervallen,
wobei, um echte R-R-Intervalle innerhalb der geordneten Liste von erfassten Intervallen zu identifizieren, der/die mindestens eine von einem Prozessor oder einer Steuerung (520) zu Folgendem konfiguriert ist/sind:
Identifizieren (418) als eines der echten R-R-Intervalle, eines jeden der erfassten Intervalle, das eine Dauer aufweist, die innerhalb eines ersten spezifizierten Schwellenwerts von entweder (i) einer Summe der Dauern von zwei unmittelbar vorhergehenden Intervallen in der geordneten Liste von erfassten Intervallen (408) oder (ii) einer Summe der Dauern von zwei unmittelbar folgenden Intervallen in der geordneten Liste von erfassten Intervallen (414) liegt; und
Identifizieren (424) als eines der echten R-R-Intervalle, jedes der erfassten Intervalle, das eine Dauer aufweist, die innerhalb eines zweiten spezifizierten Schwellenwerts eines Mittelwerts oder Medianwerts der Dauern der erfassten Intervalle liegt, die bereits als echte R-R-Intervalle identifiziert wurden, wobei der zweite spezifizierte Schwellenwert derselbe wie der erste spezifizierte Schwellenwert sein kann oder nicht; und
wobei, um eines der erfassten Intervalle, das als eines der kurzen Intervalle in der verbleibenden geordneten Liste von erfassten Intervallen klassifiziert wurde, als ein übererfasstes R-R-Intervall zu identifizieren, der/die mindestens eine von einem Prozessor oder einer Steuerung (520) dazu konfiguriert ist/sind, das erfasste Intervall als ein übererfasstes R-R-Intervall zu identifizieren, wenn alle der Folgenden zutreffen:
eine Differenz zwischen einer Dauer des erfassten Intervalls, das als eines der kurzen Intervalle klassifiziert wurde, und einer Dauer eines unmittelbar vorhergehenden erfassten Intervalls, das als eines der kurzen Intervalle klassifiziert wurde, liegt innerhalb eines dritten spezifizierten Schwellenwerts (438, 440);
eine Differenz zwischen der Dauer des erfassten Intervalls, das als eines der kurzen Intervalle klassifiziert wurde, und einer Dauer eines unmittelbar folgenden erfassten Intervalls, das als eines der langen Intervalle klassifiziert wurde, ist größer als ein vierter spezifizierter Schwellenwert (442, 444); und
die Dauer des erfassten Intervalls, das als eines der kurzen Intervalle klassifiziert wurde, ist kleiner als ein fünfter spezifizierter Schwellenwert (446).

2. Vorrichtung nach Anspruch 1, wobei für jedes erfasste Intervall, das als eines der langen Intervalle in der verbleibenden geordneten Liste von erfassten Intervallen klassifiziert ist, der/die mindestens eine von einem Prozessor oder einer Steuerung (520) dazu konfiguriert ist/sind, das erfasste Intervall als ein übererfasstes R-R-Intervall zu identifizieren, wenn ein unmittelbar vorhergehendes oder folgendes erfasstes Intervall in der verbleibenden geordneten Liste von erfassten Intervallen als eines der kurzen Intervalle klassifiziert ist und als ein übererfasstes R-R-Intervall identifiziert wurde.

3. Vorrichtung nach einem der Ansprüche 1 bis 2, wobei der/die mindestens eine von einem Prozessor oder einer Steuerung (520) ferner zu Folgendem konfiguriert ist/sind:
Bestimmen, basierend darauf, welche individuellen Intervalle als übererfasste R-R-Intervalle identifiziert werden, ob eine überwachte Herzfrequenz oder Arrhythmie-Episodenerkennung aufgrund einer Übererfassung ungenau ist und somit ignoriert oder neu berechnet werden sollte.

4. Vorrichtung nach Anspruch 3, wobei, um basierend darauf, welche einzelnen Intervalle als übererfasste R-R-Intervalle identifiziert werden, zu bestimmen, ob eine überwachte Herzfrequenz oder Arrhythmie-Episodenerkennung aufgrund von Übererfassung ungenau ist und somit ignoriert oder neu berechnet werden sollte, der/die mindestens eine von einem Prozessor oder einer Steuerung (520) zu Folgendem konfiguriert ist/sind:
Bestimmen (452) eines Übererfassungswerts basierend darauf, wie viele der erfassten Intervalle, die als eines der kurzen Intervalle klassifiziert sind, als ein übererfasstes R-R-Intervall identifiziert wurden; und
Bestimmen (454-458), basierend auf dem Übererfassungswert, ob eine überwachte Herzfrequenz oder Arrhythmie-Episodenerkennung aufgrund einer Übererfassung ungenau ist und somit ignoriert oder neu berechnet werden sollte.

5. Vorrichtung nach einem der Ansprüche 1 bis 4, wobei der/die mindestens eine von einem Prozessor oder einer Steuerung (520) ferner zu Folgendem konfiguriert ist/sind:
Kombinieren von Paaren von übererfassten R-R-Intervallen dazu, weitere echte R-R-Intervalle zu identifizieren;
Erzeugen einer korrigierten geordneten Liste von erfassten Intervallen, die echte R-R-Intervalle beinhaltet, von denen mindestens einige durch Kombinieren von Paaren von übererfassten R-R-Intervallen identifiziert werden; und
Überwachen einer Herzfrequenz und/oder auf eine Arrhythmie-Episode basierend auf der korrigierten geordneten Liste von erfassten Intervallen.

6. Vorrichtung nach einem der Ansprüche 1 bis 5, wobei der/die mindestens eine von einem Prozessor oder einer Steuerung (520) dazu konfiguriert ist/sind, die Paare von übererfassten R-R-Intervallen zu kombinieren, die korrigierte geordnete Liste von erfassten Intervallen zu erzeugen und die Herzfrequenz und/oder auf eine Arrhythmie-Episode basierend auf der korrigierten geordneten Liste von erfassten Intervallen zu überwachen, als Reaktion darauf, dass eine Bestimmung vorliegt, dass eine überwachte Herzfrequenz oder eine Arrhythmie-Episodenerkennung aufgrund von Übererfassung ungenau ist und somit ignoriert oder neu berechnet werden sollte.

7. Vorrichtung nach einem der Ansprüche 1 bis 6, ferner umfassend einen Sendeempfänger (564), der dazu konfiguriert ist, drahtlos mit einer externen Vorrichtung (554) zu kommunizieren, und wobei der/die mindestens eine von einem Prozessor oder einer Steuerung (520) ferner dazu konfiguriert ist/sind, mit dem Sendeempfänger (564) zu Folgendem zu interagieren:
Veranlassen, dass Daten, die einer Vorhofflimmern-(AF-)Episode oder einer anderen Art von Arrhythmie-Episode entsprechen, die von der Vorrichtung (501) erkannt werden, selektiv an die externe Vorrichtung (554) übertragen werden; und
Verhindern, dass Daten, die einer AF-Episode oder einer anderen Art von Arrhythmie-Episode entsprechen, die von der Vorrichtung (501) erkannt werden, aber danach von der Vorrichtung (501) als eine falsch-positive Erkennung bestimmt werden, an die externe Vorrichtung (554) übertragen werden.

8. Vorrichtung nach einem der Ansprüche 1 bis 7, wobei die Vorrichtung (501) eines der Folgenden umfasst:
eine vom Benutzer tragbare Vorrichtung;
einen implantierbaren Herzmonitor (ICM);
einen Herzschrittmacher, an dem eine oder mehrere Leitungen angebracht ist/sind;
einen leitungslosen Herzschrittmacher (LCP); oder
einen implantierbaren Kardioverter-Defibrillator (ICD).

9. Verfahren, das von einer Vorrichtung (501) oder einem System durchgeführt wird, die/das die Herzfrequenz eines Herzens eines Patienten überwacht, wobei das Verfahren Folgendes umfasst:
Erhalten (402) einer geordneten Liste von erfassten Intervallen, geordnet in der Reihenfolge, in der die Intervalle erfasst wurden, wobei die erfassten Intervalle zwischen aufeinanderfolgenden von potenziellen R-Wellen in einem Signal, das die elektrische Aktivität des Herzens eines Patienten angibt, bestimmt werden, wobei jedes der erfassten Intervalle eine jeweilige Dauer aufweist, wobei die geordnete Liste von erfassten Intervallen echte R-R-Intervalle und ein oder mehrere übererfasste R-R-Intervalle aufgrund von T-Wellen- und/oder P-Wellenübererfassung beinhaltet, wobei ein echtes R-R-Intervall ein Intervall zwischen aufeinanderfolgenden tatsächlichen R-Wellen ist und wobei ein übererfasstes R-R-Intervall ein P-R-Intervall zwischen einer übererfassten P-Welle und einer darauffolgenden tatsächlichen R-Welle, ein R-P-Intervall zwischen einer tatsächlichen R-Welle und einer darauffolgenden übererfassten P-Welle, ein T-R-Intervall zwischen einer übererfassten T-Welle und einer darauffolgenden tatsächlichen R-Welle oder ein R-T-Intervall zwischen einer tatsächlichen R-Welle und einer darauffolgenden übererfassten T-Welle ist;
Identifizieren (402-424) eines tatsächlichen R-R-Intervalls als ein echtes R-R-Intervall innerhalb der geordneten Liste von erfassten Intervallen;
Entfernen (426), aus der geordneten Liste erfasster Intervalle, eines jeden der erfassten Intervalle, das als ein echtes R-R-Intervall identifiziert wurde, um dadurch eine verbleibende geordnete Liste von erfassten Intervallen zu erzeugen;
Klassifizieren (428) individueller Intervalle in der verbleibenden geordneten Liste von erfassten Intervallen entweder als ein kurzes Intervall, wenn eine Dauer des Intervalls kürzer als die Dauer eines unmittelbar folgenden Intervalls und/oder die Dauer eines unmittelbar vorhergehenden Intervalls in der verbleibenden geordneten Liste von erfassten Intervallen ist, oder als ein langes Intervall, wenn eine Dauer des Intervalls länger als die Dauer eines unmittelbar folgenden Intervalls und/oder die Dauer eines unmittelbar vorhergehenden Intervalls in der verbleibenden geordneten Liste von erfassten Intervallen ist; und
Identifizieren (430-450) erfasster Intervalle in der verbleibenden geordneten Liste von erfassten Intervallen als ein oder mehrere übererfasste R-R-Intervalle basierend auf einer Analyse mindestens der kurzen Intervalle in der verbleibenden geordneten Liste von erfassten Intervallen,
wobei das Identifizieren echter R-R-Intervalle (404-424) innerhalb der geordneten Liste von erfassten Intervallen Folgendes umfasst:
Identifizieren (408, 410, 414, 416, 418) als eines der echten R-R-Intervalle eines jeden der erfassten Intervalle, das eine Dauer aufweist, die innerhalb eines ersten spezifizierten Schwellenwerts von entweder (i) einer Summe der Dauern von zwei unmittelbar vorhergehenden Intervallen in der geordneten Liste von erfassten Intervallen oder (ii) einer Summe der Dauern von zwei unmittelbar folgenden Intervallen in der geordneten Liste von erfassten Intervallen liegt; und
Identifizieren (422, 424) als eines der echten R-R-Intervalle eines jeden der erfassten Intervalle, das eine Dauer aufweist, die innerhalb eines zweiten spezifizierten Schwellenwerts eines Mittelwerts oder Medianwerts der Dauern der erfassten Intervalle liegt, die bereits als echte R-R-Intervalle identifiziert wurden, wobei der zweite spezifizierte Schwellenwert derselbe wie der erste spezifizierte Schwellenwert sein kann oder nicht; und
wobei das Identifizieren (430, 450) eines oder mehrerer übererfasster R-R-Intervalle für mindestens eines der erfassten Intervalle, das als eines der kurzen Intervalle in der verbleibenden geordneten Liste von erfassten Intervallen klassifiziert ist, Identifizieren des erfassten Intervalls als ein übererfasstes R-R-Intervall umfasst, wenn alle der Folgenden zutreffen:
(438, 440) eine Differenz zwischen einer Dauer des erfassten Intervalls, das als eines der kurzen Intervalle klassifiziert wurde, und einer Dauer eines unmittelbar vorhergehenden erfassten Intervalls, das als eines der kurzen Intervalle klassifiziert wurde, liegt innerhalb eines dritten spezifizierten Schwellenwerts;
(442, 444) eine Differenz zwischen der Dauer des erfassten Intervalls, das als eines der kurzen Intervalle klassifiziert wurde, und einer Dauer eines unmittelbar folgenden erfassten Intervalls, das als eines der langen Intervalle klassifiziert wurde, ist größer als ein vierter spezifizierter Schwellenwert; und
(446) die Dauer des erfassten Intervalls, das als eines der kurzen Intervalle klassifiziert wurde, ist kleiner als ein fünfter spezifizierter Schwellenwert.

10. Verfahren nach Anspruch 9, wobei das Identifizieren (430-450) eines oder mehrerer übererfasster R-R-Intervalle basierend auf Ergebnissen des Klassifizierens eines jeden der Intervalle in der verbleibenden geordneten Liste von erfassten Intervallen entweder als eines der kurzen Intervalle oder eines der langen Intervalle ferner Folgendes umfasst:
für jedes erfasste Intervall, das als eines der langen Intervalle klassifiziert ist, in der verbleibenden geordneten Liste von erfassten Intervallen, Identifizieren des erfassten Intervalls als ein übererfasstes R-R-Intervall, wenn ein unmittelbar vorhergehendes oder folgendes erfasstes Intervall, das als eines der kurzen Intervalle klassifiziert ist, als ein übererfasstes R-R-Intervall identifiziert wurde.

11. Verfahren nach einem der Ansprüche 9 bis 10, ferner umfassend:
Bestimmen (452) eines Übererfassungswerts basierend darauf, wie viele der erfassten Intervalle, die als eines der kurzen Intervalle klassifiziert sind, als ein übererfasstes R-R-Intervall identifiziert wurden; und
Bestimmen (454-456), dass eine erkannte AF-Episode oder eine andere Art von Arrhythmie-Episode eine falsch-positive Erkennung war, wenn der Übererfassungswert einen spezifizierten Übererfassungsschwellenwert überschreitet.

12. Verfahren nach Anspruch 11, wobei:
der spezifizierte Übererfassungsschwellenwert einen spezifizierten Prozentsatzschwellenwert umfasst;
das Bestimmen (452) des Übererfassungswerts Bestimmen dessen umfasst, welcher Prozentsatz aller erfassten Intervalle, die als eines der kurzen Intervalle klassifiziert sind, als ein übererfasstes R-R-Intervall identifiziert wurden; und
das Bestimmen (454-456), dass eine erkannte AF-Episode oder eine andere Art von Arrhythmie-Episode eine falsch-positive Erkennung war, auftritt, wenn der bestimmte Prozentsatz den spezifizierten Prozentsatzschwellenwert überschreitet.

## Revendications

1. Dispositif (501), comprenant :
une ou plusieurs électrodes (500, 502, 504, 506, 508, 510) ;
un circuit de détection (544) couplé à l'une ou plusieurs électrodes (500, 502, 504, 506, 508, 510) et configuré pour obtenir un signal indicatif d'une activité électrique d'un cœur de patient ; et
au moins un parmi un processeur ou un dispositif de commande (520) configuré pour :
obtenir une liste ordonnée d'intervalles détectés, ordonnés dans l'ordre où les intervalles ont été détectés, dans lequel les intervalles détectés sont déterminés entre des ondes R potentielles consécutives dans le signal indicatif de l'activité électrique du cœur de patient, dans lequel chacun des intervalles détectés a une durée respective, dans lequel la liste ordonnée d'intervalles détectés inclut des intervalles R-R réels et un ou plusieurs intervalles R-R surdétectés en raison d'une surdétection d'onde T et/ou d'onde P, dans lequel un intervalle R-R réel est un intervalle entre des ondes R réelles consécutives, et dans lequel un intervalle R-R surdétecté est un intervalle P-R entre une onde P surdétectée et une onde R réelle consécutive, un intervalle R-P entre une onde R réelle et une onde P surdétectée consécutive, un intervalle T-R entre une onde T surdétectée et une onde R réelle consécutive, ou un intervalle R-T entre une onde R réelle et une onde T surdétectée consécutive ;
identifier des intervalles R-R réels en tant qu'intervalles R-R réels à l'intérieur de la liste ordonnée d'intervalles détectés ;
retirer de la liste ordonnée d'intervalles détectés chacun des intervalles détectés qui a été identifié comme étant un intervalle R-R réel, pour produire ainsi une liste ordonnée restante d'intervalles détectés ;
classer des intervalles individuels, dans la liste ordonnée restante d'intervalles détectés, soit comme un intervalle court lorsqu'une durée de l'intervalle est plus courte que la durée d'un intervalle suivant immédiatement et/ou la durée d'un intervalle précédant immédiatement dans la liste ordonnée restante d'intervalles détectés, soit comme un intervalle long lorsqu'une durée de l'intervalle est plus longue que la durée d'un intervalle suivant immédiatement et/ou la durée d'un intervalle précédant immédiatement dans la liste ordonnée restante d'intervalles détectés ; et
identifier des intervalles détectés dans la liste ordonnée restante d'intervalles détectés en tant qu'intervalles R-R surdétectés sur la base d'une analyse d'au moins les intervalles courts dans la liste ordonnée restante d'intervalles détectés,
dans lequel afin d'identifier des intervalles R-R réels à l'intérieur de la liste ordonnée d'intervalles détectés l'au moins un parmi un processeur ou un dispositif de commande (520) est configuré pour :
identifier (418) en tant que dit intervalle R-R réel, chaque dit intervalle détecté ayant une durée qui est dans la limite d'un premier seuil spécifié de soit (i) une somme des durées de deux intervalles précédant immédiatement dans la liste ordonnée d'intervalles détectés (408), soit (ii) une somme des durées de deux intervalles suivant immédiatement dans la liste ordonnée d'intervalles détectés (414) ; et
identifier (424) en tant que dit intervalle R-R réel, chaque dit intervalle détecté ayant une durée qui est dans la limite d'un deuxième seuil spécifié d'une moyenne ou d'une médiane des durées des intervalles détectés déjà identifiés en tant qu'intervalles R-R réels, dans lequel le deuxième seuil spécifié peut ou ne peut pas être le même que le premier seuil spécifié ; et
dans lequel afin d'identifier un dit intervalle détecté, qui a été classé comme un dit intervalle court dans la liste ordonnée restante d'intervalles détectés, en tant qu'intervalle R-R surdétecté, l'au moins un parmi un processeur ou un dispositif de commande (520) est configuré pour identifier l'intervalle détecté en tant qu'intervalle R-R surdétecté lorsque tout ce qui suit est vrai :
une différence entre une durée de l'intervalle détecté qui a été classé comme un dit intervalle court et une durée d'un intervalle détecté précédant immédiatement qui a été classé comme un dit intervalle court est dans la limite d'un troisième seuil spécifié (438, 440) ;
une différence entre la durée de l'intervalle détecté qui a été classé comme un dit intervalle court et une durée d'un intervalle détecté suivant immédiatement qui a été classé comme un dit intervalle long est supérieure à un quatrième seuil spécifié (442, 444) ; et
la durée de l'intervalle détecté qui a été classé comme un dit intervalle court est inférieure à un cinquième seuil spécifié (446).

2. Dispositif de la revendication 1, dans lequel pour chaque intervalle détecté classé comme un dit intervalle long, dans la liste ordonnée restante d'intervalles détectés, l'au moins un parmi un processeur ou un dispositif de commande (520) est configuré pour identifier l'intervalle détecté en tant qu'intervalle R-R surdétecté si un intervalle détecté précédant ou suivant immédiatement, dans la liste ordonnée restante d'intervalles détectés, est classé comme un dit intervalle court et a été identifié comme un intervalle R-R surdétecté.

3. Dispositif de l'une quelconque des revendications 1 à 2, dans lequel l'au moins un parmi un processeur ou un dispositif de commande (520) est en outre configuré(s) pour :
déterminer, sur la base des intervalles individuels qui sont identifiés en tant qu'intervalles R-R surdétectés, si un rythme cardiaque surveillé ou une détection d'épisode arythmique est inexact en raison d'une surdétection et devrait donc être ignoré ou recalculé.

4. Dispositif de la revendication 3, dans lequel afin de déterminer, sur la base des intervalles individuels qui sont identifiés en tant qu'intervalles R-R surdétectés, si un rythme cardiaque surveillé ou une détection d'épisode arythmique est inexact en raison d'une surdétection et devrait donc être ignoré ou recalculé, l'au moins un parmi un processeur ou un dispositif de commande (520) est configuré pour :
déterminer (452) un score de surdétection sur la base du nombre des intervalles détectés classés comme un dit intervalle court qui ont été identifiés comme étant un intervalle R-R surdétecté ; et
déterminer (454-458), sur la base du score de surdétection, si un rythme cardiaque surveillé ou une détection d'épisode arythmique est inexact en raison d'une surdétection et devrait donc être ignoré ou recalculé.

5. Dispositif selon l'une quelconque des revendications 1 à 4, dans lequel l'au moins un parmi un processeur ou un dispositif de commande (520) est en outre configuré(s) pour :
combiner des paires d'intervalles R-R surdétectés pour identifier d'autres intervalles R-R réels ;
produire une liste ordonnée corrigée d'intervalles détectés incluant des intervalles R-R réels, dont au moins certains sont identifiés en combinant des paires d'intervalles R-R surdétectés ; et
surveiller le rythme cardiaque et/ou un épisode arythmique sur la base de la liste ordonnée corrigée d'intervalles détectés.

6. Dispositif selon l'une quelconque des revendications 1 à 5, dans lequel l'au moins un parmi un processeur ou un dispositif de commande (520) est configuré pour combiner lesdites paires d'intervalles R-R surdétectés, produire la liste ordonnée corrigée d'intervalles détectés, et surveiller le rythme cardiaque et/ou un épisode arythmique sur la base de la liste ordonnée corrigée d'intervalles détectés, en réponse au fait qu'il y a une détermination qu'un rythme cardiaque surveillé ou une détection d'épisode arythmique est inexact en raison d'une surdétection et devrait donc être ignoré ou recalculé.

7. Dispositif selon l'une quelconque des revendications 1 à 6, comprenant en outre un émetteur-récepteur (564) configuré pour communiquer sans fil avec un dispositif externe (554), et dans lequel l'au moins un parmi un processeur ou un dispositif de commande (520) est en outre configuré pour interagir avec l'émetteur-récepteur (564) pour :
amener des données correspondant à un épisode de fibrillation auriculaire (FA) ou un autre type d'épisode arythmique qui est détecté par le dispositif (501) à être transmises sélectivement au dispositif externe (554) ; et
empêcher des données correspondant à un épisode de FA ou un autre type d'épisode arythmique qui est détecté par le dispositif (501), mais est ensuite déterminé par le dispositif (501) comme étant une détection faussement positive, d'être transmises au dispositif externe (554).

8. Dispositif selon l'une quelconque des revendications 1 à 7, dans lequel le dispositif (501) comprend l'un parmi ce qui suit :
un dispositif portable par l'utilisateur ;
un moniteur cardiaque insérable (ICM) ;
un stimulateur cardiaque auquel une ou plusieurs dérivations sont attachées ;
un stimulateur cardiaque sans fil (LCP) ; ou
un défibrillateur automatique implantable (ICD).

9. Procédé réalisé par un dispositif (501) ou système qui surveille un rythme cardiaque d'un cœur de patient, le procédé comprenant :
l'obtention (402) d'une liste ordonnée d'intervalles détectés, ordonnés dans l'ordre où les intervalles ont été détectés, dans lequel les intervalles détectés sont déterminés entre des ondes R potentielles consécutives dans un signal indicatif de l'activité électrique du cœur de patient, dans lequel chacun des intervalles détectés a une durée respective, dans lequel la liste ordonnée d'intervalles détectés inclut des intervalles R-R réels et un ou plusieurs intervalles R-R surdétectés en raison d'une surdétection d'onde T et/ou d'onde P, dans lequel un intervalle R-R réel est un intervalle entre des ondes R réelles consécutives, et dans lequel un intervalle R-R surdétecté est un intervalle P-R entre une onde P surdétectée et une onde R réelle consécutive, un intervalle R-P entre une onde R réelle et une onde P surdétectée consécutive, un intervalle T-R entre une onde T surdétectée et une onde R réelle consécutive, ou un intervalle R-T entre une onde R réelle et une onde T surdétectée consécutive ;
l'identification (402-424) d'un intervalle R-R réel en tant qu'intervalle R-R réel à l'intérieur de la liste ordonnée d'intervalles détectés ;
le retrait (426), de la liste ordonnée d'intervalles détectés, de chacun des intervalles détectés qui a été identifié comme étant un intervalle R-R réel, pour produire ainsi une liste ordonnée restante d'intervalles détectés ;
la classification (428) d'intervalles individuels dans la liste ordonnée restante d'intervalles détectés soit comme un intervalle court lorsqu'une durée de l'intervalle est plus courte que la durée d'un intervalle suivant immédiatement et/ou la durée d'un intervalle précédant immédiatement dans la liste ordonnée restante d'intervalles détectés, soit comme un intervalle long lorsqu'une durée de l'intervalle est plus longue que la durée d'un intervalle suivant immédiatement et/ou la durée d'un intervalle précédant immédiatement dans la liste ordonnée restante d'intervalles détectés ; et
l'identification (430-450) d'intervalles détectés dans la liste ordonnée restante d'intervalles détectés en tant qu'un ou plusieurs intervalles R-R surdétectés sur la base d'une analyse d'au moins les intervalles courts dans la liste ordonnée restante d'intervalles détectés,
dans lequel l'identification d'intervalles R-R réels (404-424) à l'intérieur de la liste ordonnée d'intervalles détectés comprend :
l'identification (408, 410, 414, 416, 418) en tant que dit intervalle R-R réel, de chaque dit intervalle détecté ayant une durée qui est dans la limite d'un premier seuil spécifié de soit (i) une somme des durées de deux intervalles précédant immédiatement dans la liste ordonnée d'intervalles détectés, soit (ii) une somme des durées de deux intervalles suivant immédiatement dans la liste ordonnée d'intervalles détectés ; et
l'identification (422, 424) en tant que dit intervalle R-R réel, de chaque dit intervalle détecté ayant une durée qui est dans la limite d'un deuxième seuil spécifié d'une moyenne ou d'une médiane des durées des intervalles détectés déjà identifiés en tant qu'intervalles R-R réels, dans lequel le deuxième seuil spécifié peut ou ne peut pas être le même que le premier seuil spécifié ; et
dans lequel l'identification (430, 450) d'un ou plusieurs intervalles R-R surdétectés comprend, pour au moins un dit intervalle détecté classé comme un dit intervalle court dans la liste ordonnée restante d'intervalles détectés, l'identification de l'intervalle détecté en tant qu'intervalle R-R surdétecté lorsque tout ce qui suit est vrai :
(438, 440) une différence entre une durée de l'intervalle détecté qui a été classé comme un dit intervalle court et une durée d'un intervalle détecté précédant immédiatement qui a été classé comme un dit intervalle court est dans la limite d'un troisième seuil spécifié ;
(442, 444) une différence entre la durée de l'intervalle détecté qui a été classé comme un dit intervalle court et une durée d'un intervalle détecté suivant immédiatement qui a été classé comme un dit intervalle long est supérieure à un quatrième seuil spécifié ; et
(446) la durée de l'intervalle détecté qui a été classé comme un dit intervalle court est inférieure à un cinquième seuil spécifié.

10. Procédé selon la revendication 9, dans lequel l'identification (430-450) d'un ou plusieurs intervalles R-R surdétectés sur la base de résultats de la classification de chacun des intervalles dans la liste ordonnée restante d'intervalles détectés soit comme un dit intervalle court soit comme un dit intervalle long, comprend en outre :
pour chaque intervalle détecté classé comme un dit intervalle long, dans la liste ordonnée restante d'intervalles détectés, l'identification de l'intervalle détecté en tant qu'intervalle R-R surdétecté si un intervalle détecté précédant ou suivant immédiatement classé comme un dit intervalle court a été identifié comme un intervalle R-R surdétecté.

11. Procédé selon l'une quelconque des revendications 9 à 10, comprenant en outre :
la détermination (452) d'un score de surdétection sur la base du nombre des intervalles détectés classés comme un dit intervalle court qui ont été identifiés comme étant un intervalle R-R surdétecté ; et
la détermination (454-456) qu'un épisode de FA détecté ou un autre type d'épisode arythmique était une détection faussement positive lorsque le score de surdétection dépasse un seuil de surdétection spécifié.

12. Procédé selon la revendication 11, dans lequel :
le seuil de surdétection spécifié comprend un seuil de pourcentage spécifié ;
la détermination (452) du score de surdétection comprend la détermination de quel pourcentage de la totalité des intervalles détectés classés comme un dit intervalle court a été identifié comme étant un intervalle R-R surdétecté ; et
la détermination (454-456) qu'un épisode de FA détecté ou un autre type d'épisode arythmique était une détection faussement positive se produit lorsque le pourcentage déterminé dépasse le seuil de pourcentage spécifié.
